(19) Europäisches Patentamt
European Patent Office
Office européen des brevets

(11) EP 3 036 241 B1

(12) EUROPÄISCHE PATENTSCHRIFT

(45) Veröffentlichungstag und Bekanntmachung des Hinweises auf die Patenterteilung:
**04.10.2017 Patentblatt 2017/40**

(21) Anmeldenummer: **14744772.6**

(22) Anmeldetag: **25.07.2014**

(51) Int Cl.:
***C07F 1/00*** *(2006.01)* ***C09K 11/06*** *(2006.01)*
***H01L 51/50*** *(2006.01)* ***H05B 33/10*** *(2006.01)*

(86) Internationale Anmeldenummer:
**PCT/EP2014/002035**

(87) Internationale Veröffentlichungsnummer:
**WO 2015/039715 (26.03.2015 Gazette 2015/12)**

# (54) METALLKOMPLEXE

METAL COMPLEXES

COMPLEXES MÉTALLIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.08.2013 DE 102013013876**

(43) Veröffentlichungstag der Anmeldung:
**29.06.2016 Patentblatt 2016/26**

(73) Patentinhaber: **Merck Patent GmbH**
**64293 Darmstadt (DE)**

(72) Erfinder:
- **BECKER, Heinrich**
**64372 Ober-Ramstadt (DE)**
- **VOGES, Frank**
**67098 Bad Duerkheim (DE)**

(56) Entgegenhaltungen:
**FR-A1- 2 974 505 JP-A- 2007 063 489**

- **DATABASE CA [Online] CHEMICAL ABSTRACTS SERVICE, COLUMBUS, OHIO, US; FRIEDRICH, A. ET AL: "Chelates of 8-quinolinol derivatives. VIII. Acid and complex stability constants of alkyl and alkenyl substituted 8-quinolinols", XP002730670, gefunden im STN Database accession no. 1986:413052**
- **KOLOBIELSKI, MARJAN: "The synthesis of substituted 8-quinolinols", JOURNAL OF HETEROCYCLIC CHEMISTRY, Bd. 3, Nr. 3, 1966, Seiten 275-277, XP002730671, ISSN: 0022-152X, DOI: 10.1002/JHET.5570030308**
- **THOMPSON, MICHAEL ET AL: "Ultraviolet photoelectron spectroscopy and oxidative electrochemistry of 8-hydroxyquinoline and its derivatives", ANALYTICA CHIMICA ACTA, Bd. 119, Nr. 1, 1980, Seiten 179-185, XP002730672, ISSN: 0003-2670, DOI: 10.1016/S0003-2670(00)00047-7**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 036 241 B1

Printed by Jouve, 75001 PARIS (FR)

**Beschreibung**

Die vorliegende Erfindung betrifft Metallkomplexe, Zusammensetzungen und Formulierungen enthaltend diese, sowie Vorrichtungen, insbesondere elektronische Vorrichtungen enthaltend die Metallkomplexe und Zusammensetzungen, deren Herstellung und Zwischenstufen der erfindungsgemäßen Metallkomplexe.

Elektronische Vorrichtungen, welche organische, metallorganische und/oder polymere Halbleiter enthalten, gewinnen zunehmend an Bedeutung, wobei diese aus Kostengründen und aufgrund ihrer Leistungsfähigkeit in vielen kommerziellen Produkten eingesetzt werden. Als Beispiele seien hier Ladungstransportmaterialien auf organischer Basis (z.B. Lochtransporter auf Triarylamin-Basis) in Kopiergeräten, organischen oder polymeren Leuchtdioden (OLEDs oder PLEDs) und in Anzeige- und Displayvorrichtungen oder organische Photorezeptoren in Kopierern genannt. Organische Solarzellen (O-SC), organische FeldeffektTransistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Viele dieser elektronischen Vorrichtungen weisen unabhängig von dem jeweiligen Verwendungszweck folgenden allgemeinen Schichtaufbau auf, der für die jeweilige Anwendung angepasst werden kann:

(1) Substrat,
(2) Elektrode, häufig metallisch oder anorganisch, aber auch aus organischen bzw. polymeren, leitfähigen Materialien,
(3) Ladungsinjektionsschicht/en bzw. Zwischenschicht/en, beispielsweise zum Ausgleich von Unebenheiten der Elektrode ("planarisation layer"), häufig aus einem leitfähigen, dotierten Polymer,
(4) Organische Halbleiter,
(5) evtl. weitere Ladungstransport-, Ladungsinjektions- bzw. Ladungsblockierschichten,
(6) Gegenelektrode, Materialien wie unter (2) genannt,
(7) Verkapselung.

Die obige Anordnung stellt den allgemeinen Aufbau einer organischen, elektronischen Vorrichtung dar, wobei verschiedene Schichten zusammengefasst werden können, so dass im einfachsten Fall eine Anordnung aus zwei Elektroden resultiert, zwischen denen sich eine organische Schicht befindet. Die organische Schicht erfüllt in diesem Fall alle Funktionen, einschließlich der Emission von Licht im Fall von OLEDs. Ein derartiges System ist beispielsweise in der WO 90/13148 A1 auf der Basis von Poly-(p-phenylenen) beschrieben. JP 2007063489 A offenbart Verbindugen zur Verwendung in elektronischen Vorrichtungen. Zur Verbesserung der Effizienz und der Lebensdauer elektronischer Vorrichtungen können in organischen Schichten, insbesondere in elektronenleitenden Schichten, wie beispielsweise in Elektronentransport-, Elektroneninjektions-, Lochblockier- und Emissiosschichten, Metall-Hydroxychinolin-Verbindungen eingesetzt werden. Nachteilig ist jedoch, dass die bisher eingesetzten Hydroxychinolin-Verbindungen mutagene Wirkungen aufweisen. Weiterhin zeigen die aus dem Stand der Technik bekannten Verbindungen, die meist aufgedampft werden, eine schlechte oder nur mäßige Prozessierbarkeit auf.

Aufgabe der vorliegenden Erfindung ist daher die Bereitstellung neuer Verbindungen und neuer Vorrichtungen, welche die bekannten Nachteile aus dem Stand der Technik beseitigen. Insbesondere sollen die Verbindungen sowie die Vorrichtungen enthaltend diese Verbindungen umweltfreundlicher bzw. weniger gesundheitsgefährdend sein. Insbesondere stellt die Bereitstellung von Verbindungen mit elektronentransportierenden Eigenschaften mit einer geringen Mutagenität oder einer geringen kanzerogenen Wirkung eine Aufgabe der vorliegenden Erfindung dar.

Weitere Eigenschaften der Vorrichtung, insbesondere der hier gegebenenfalls eingesetzten Lochinjektionsmaterialien, Lochtransportmaterialien, Lochblockiermaterialien, Elektroneninjektionsmaterialien, Elektronenblockiermaterialien und/oder Emittermaterialien sollten durch die Elektronentransportmaterialien nicht oder nur unwesentlich beeinträchtig werden, wie beispielsweise Effizienz, Betriebsspannung, Lebensdauer, Farbkoordinaten und/oder Farbreinheit, d. h. Breite der Emissionsbande. Eine weitere Aufgabe kann darin gesehen werden, elektronische Vorrichtungen mit einer ausgezeichneten Leistungsfähigkeit möglichst kostengünstig und in konstanter Qualität bereitzustellen. Weiterhin sollen die neuen Materialien besser prozessierbar sein und sich somit besser für eine Massenproduktion eignen.

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschenderweise wurde gefunden, dass die weiter unten näher beschriebenen Verbindungen, Zusammensetzungen, Formulierungen und Vorrichtungen diese sowie weitere nicht explizit genannte Aufgaben, die jedoch aus den hierin einleitend diskutierten Zusammenhängen ohne Weiteres ableitbar oder erschließbar sind, lösen.

Die vorliegende Erfindung betrifft daher eine Verbindung der allgemeinen Formel (1)

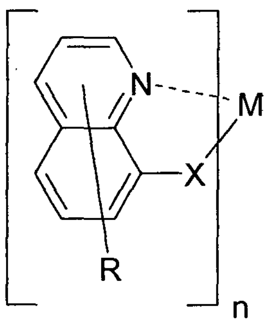

Formel (1)

wobei

M ist

X ist S oder O, bevorzugt O;

R eine verzweigte oder cyclische Alkyl- oder Alkoxygruppe mit 3 bis 40 C-Atomen, die jeweils mit einem oder mehreren Resten $R^a$ substituiert sein kann, wobei eine oder mehrere nicht benachbarte $CH_2$-Gruppen durch $R^aC{=}CR^a$, C=C , $Si(R^a)_2$, $Ge(R^a)_2$, $Sn(R^a)_2$, C=O, C=S, C=Se, $C{=}NR^a$, $P(=O)(R^a)$, SO, $SO_2$ $NR^a$, O, S oder $CONR^a$ ersetzt sein können und wobei ein oder mehrere H-Atome durch D, F, Cl, Br, I, CN oder $NO_2$ ersetzt sein können, oder ein aromatisches oder heteroaromatisches Ringsystem, bevorzugt ein aromatisches Ringsystem, mit 5 bis 60 aromatischen Ringatomen, das zumindest in einer ortho-Position in Bezug auf die Bindungsstelle an den Chinolinring einen Rest $R^b$ aufweist und gegebenenfalls durch einen oder mehrere Reste $R^a$ substituiert sein kann, wobei der Rest R bevorzugt kein Ringsystem zusammen mit dem Chinolinring bildet;

$R^a$ bei jedem Auftreten gleich oder verschieden H, D oder eine Alkylgruppe mit 1 bis 20 C-Atomen, ein aromatisches Ringsystem mit 6 bis 60 C-Ring-Atomen oder ein heteroaromatisches Ringsystem mit 1 bis 60 C-Ring-Atomen ist, in dem auch H-Atome durch D oder F ersetzt sein können; dabei können zwei oder mehrere benachbarte Substituenten $R^a$ auch miteinander ein mono- oder polycyclisches, aliphatisches, aromatisches oder heteroaromatisches Ringsystem bilden wobei bevorzugt ist, wenn zwei oder mehrere benachbarte Reste $R^a$ keinen Ringschluss bilden;

$R^b$ bei jedem Auftreten gleich oder verschieden eine Alkylgruppe mit 1 bis 20 C-Atomen, ein aromatisches Ringsystem mit 6 bis 60 C-Ring-Atomen oder heteroaromatisches Ringsystem mit 1 bis 60 C-Ring-Atomen, wobei H-Atome durch D oder F ersetzt sein können; dabei können zwei oder mehrere benachbarte Substituenten $R^b$ auch miteinander ein mono- oder polycyclisches, aliphatisches oder aromatisches Ringsystem bilden, wobei bevorzugt ist, wenn zwei oder mehrere benachbarte Reste $R^b$ keinen Ringschluss bilden

n ist 1;

mit der Maßgabe, dass wenn R ein aromatisches Ringsystem ist, das in nur einer ortho-Position zum Chinolinring einen Rest $R^b$ aufweist, der Rest $R^b$ mindestens 2 C-Atome, bevorzugt mindestens 3 C-Atome und ganz bevorzugt mindestens 4 C-Atome aufweist, und wobei R an Position 2 an den Chinolinring gebunden ist. Die erfindungsgemäßen Verbindungen weisen, verglichen mit Verbindungen aus dem Stand der Technik, in standardisierten Verfahren eine geringere mutagene Wirkung auf. Es wird angenommen, dass die erfindungsgemäßen Reste R, die sterisch anspruchsvoll sind und aus der Ebene, die durch das Chinolinringsystem aufgespannt werden, herausragen, die mutagene Wirkung verhindern. Andere Eigenschaften der erfindungsgemäßen Verbindungen, bedingt durch die speziellen Reste R, können alternativ oder zusätzlich ursächlich für die genannten vorteilhaften technischen Effekte sein.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

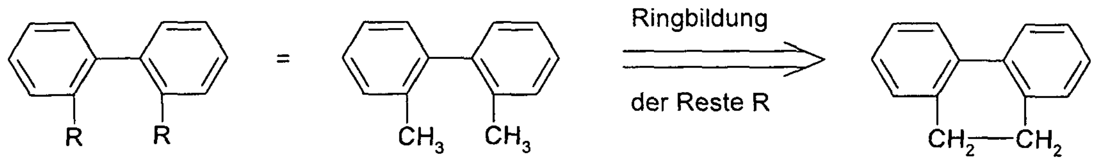


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

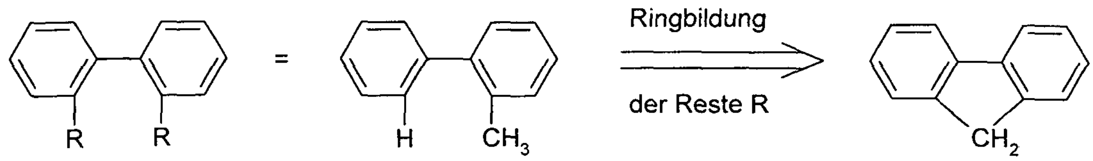


Benachbarte Reste im Sinne der vorliegenden Erfindung sind nicht nur dann benachbart, wenn die Atome, an welche die Reste binden durch nur eine oder zwei chemische Bindungen voneinander getrennt sind, sondern auch dann, wenn die Atome an welche die Reste binden durch mehr als 2 chemische Bindungen voneinander getrennt sind, solange die Reste sich zueinander noch in räumlicher Nachbarschaft befinden.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl oder Terphenyl, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 60 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Dotanden sind im Allgemeinen diejenigen Materialien, deren Anteil im System, beispielsweise in einer Schicht, der kleinere ist und das Matrixmaterial oder die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden. Die kann der Fall sein, wenn Mixed-Matrix-Systeme verwendet werden.

Unter einem n-Dotanden wird vorliegend eine organische oder anorganische Verbindung verstanden, die in der Lage ist Elektronen abzugeben (Elektronendonator), d.h. eine Verbindung, die als Reduktionsmittel wirkt.

Vom Begriff fluoreszierender Emitter (auch fluoreszierender Dotand genannt) sind typischerweise Verbindungen umfasst, bei denen Lichtemission durch einen spinerlaubten Übergang aus einem SingulettZustand erfolgt

Vom Begriff phosphoreszierender Emitter (auch phosphoreszierender Dotand genannt) sind typischerweise Verbindungen umfasst, bei denen die Lichtemission durch einen spinverbotenen Übergang erfolgt, beispielsweise einen Übergang aus einem Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen.

Bevorzugt ist weiterhin, dass R eine verzweigte Alkylgruppe oder eine cyclische Alkylgruppe ist, die keine Heteroatome umfasst.

Insbesondere bevorzugt ist, dass R mindestens 4 Kohlenstoffatome umfasst.

Weiterhin bevorzugt ist, dass R 4 bis 10 Atome aufweist, ausgewählt aus der Gruppe bestehend aus C, N, S und/oder O.

In einer bevorzugten Ausführungsform der vorliegenden Erfindung ist der Rest R eine verzweigte Alkylgruppe mit 4 bis 40 C-Atomen, bevorzugt mit 4 bis 30 C-Atomen, ganz bevorzugt mit 4 bis 20 C-Atomen, ganz besonders bevorzugt mit 4 bis 10 C-Atomen und insbesondere bevorzugt mit 4 bis 6 C-Atomen ist, eine cyclische Alkylgruppe mit 3 bis 40 C-Atomen, bevorzugt mit 3 bis 30 C-Atomen, ganz bevorzugt mit 3 bis 20 C-Atomen, ganz besonders bevorzugt mit 3 bis 15 C-Atomen und insbesondere bevorzugt mit 6 bis 12 C-Atomen ist, wobei die Gruppen jeweils mit einem oder mehreren Resten $R^a$ substituiert sein können und wobei ein oder mehrere H-Atome durch D, F, Cl, Br, I, CN oder $NO_2$ ersetzt sein können, oder ein aromatisches Ringsystem mit 5 bis 60 aromatischen Ringatomen, das zumindest in einer ortho-Position in Bezug auf die Bindungsstelle an den Chinolinring einen Rest $R^b$ aufweist und gegebenenfalls durch einen oder mehrere Reste $R^a$ substituiert sein kann, wobei bevorzugt keiner der Reste R, $R^a$ oder $R^b$ mit dem Chinolinring ein Ringsystem bildet und wobei die oben angegebene Maßgabe für das in ortho-Position substituierte Ringsystem gilt.

In einer ganz bevorzugten Ausführungsform der vorliegenden Erfindung ist der Rest R eine verzweigte Alkylgruppe mit 4 bis 40 C-Atomen, bevorzugt mit 4 bis 30 C-Atomen, ganz bevorzugt mit 4 bis 20 C-Atomen, ganz besonders bevorzugt mit 4 bis 10 C-Atomen und insbesondere bevorzugt mit 4 bis 6 C-Atomen ist oder eine cyclische Alkylgruppe

mit 3 bis 40 C-Atomen, bevorzugt mit 3 bis 30 C-Atomen, ganz bevorzugt mit 3 bis 20 C-Atomen, ganz besonders bevorzugt mit 3 bis 15 C-Atomen und insbesondere bevorzugt mit 6 bis 12 C-Atomen ist, wobei die Gruppen jeweils mit einem oder mehreren Resten $R^a$ substituiert sein können und wobei ein oder mehrere H-Atome durch D, F, Cl, Br, I, CN oder $NO_2$ ersetzt sein können, wobei bevorzugt keiner der Reste R, $R^a$ oder $R^b$ mit dem Chinolinring ein Ringsystem bildet.

In einer weiteren ganz bevorzugten Ausführungsform der vorliegenden Erfindung ist der Rest R eine cyclische Alkylgruppe mit 3 bis 40 C-Atomen, bevorzugt mit 3 bis 30 C-Atomen, ganz bevorzugt mit 3 bis 20 C-Atomen, ganz besonders bevorzugt mit 3 bis 15 C-Atomen und insbesondere bevorzugt mit 6 bis 12 C-Atomen ist, wobei die Gruppen jeweils mit einem oder mehreren Resten $R^a$ substituiert sein können und wobei ein oder mehrere H-Atome durch D, F, Cl, Br, I, CN oder $NO_2$ ersetzt sein können, wobei bevorzugt keiner der Reste R, $R^a$ oder $R^b$ mit dem Chinolinring ein Ringsystem bildet.

In einer ganz besonders bevorzugten Ausführungsform der vorliegenden Erfindung ist der Rest R eine verzweigte Alkylgruppe mit 4 bis 40 C-Atomen, bevorzugt mit 4 bis 30 C-Atomen, ganz bevorzugt mit 4 bis 20 C-Atomen, ganz besonders bevorzugt mit 4 bis 10 C-Atomen und insbesondere bevorzugt mit 4 bis 6 C-Atomen ist, wobei die Gruppen jeweils mit einem oder mehreren Resten $R^a$ substituiert sein können und wobei ein oder mehrere H-Atome durch D, F, Cl, Br, I, CN oder $NO_2$ ersetzt sein können, wobei bevorzugt keiner der Reste R, $R^a$ oder $R^b$ mit dem Chinolinring ein Ringsystem bildet.

In einer insbesondere bevorzugten Ausführungsform der vorliegenden Erfindung ist der Rest R eine verzweigte Alkylgruppe mit 4 bis 40 C-Atomen, bevorzugt mit 4 bis 30 C-Atomen, ganz bevorzugt mit 4 bis 20 C-Atomen, ganz besonders bevorzugt mit 4 bis 10 C-Atomen und insbesondere bevorzugt mit 4 bis 6 C-Atomen ist, wobei die Gruppen jeweils mit einem oder mehreren Resten $R^a$ substituiert sein können und wobei ein oder mehrere H-Atome durch D, F, Cl, Br, I, CN oder $NO_2$ ersetzt sein können, wobei bevorzugt keiner der Reste R, $R^a$ oder $R^b$ mit dem Chinolinring ein Ringsystem bildet.

In einer noch bevorzugteren Ausführungsform der vorliegenden Erfindung ist der Rest R eine verzweigte Alkylgruppe mit 4 bis 40 C-Atomen, bevorzugt mit 4 bis 30 C-Atomen, ganz bevorzugt mit 4 bis 20 C-Atomen, ganz besonders bevorzugt mit 4 bis 10 C-Atomen und insbesondere bevorzugt mit 4 bis 6 C-Atomen ist, wobei die Gruppen nicht weiter substituiert sind und wobei bevorzugt keiner der Reste R, $R^a$ oder $R^b$ mit dem Chinolinring ein Ringsystem bildet.

In einer bevorzugten Ausführungsform der vorliegenden Erfindung ist $R^a$ in der Verbindung der Formel (1) kein heteroaromatisches Ringsystem.

In einer weiteren bevorzugten Ausführungsform der vorliegenden Erfindung ist $R^b$ in der Verbindung der Formel (1) kein heteroaromatisches Ringsystem.

Es ist ganz bevorzugt, wenn die Verbindung der Formel (1) neben dem Chinolin überhaupt keine weiteren heteroaromatischen Ringsysteme enthält. Dies hat den technischen Effekt, dass die Leistungsdaten elektrolumineszierender Vorrichtungen besser sind, die Prozessierbarkeit der Verbindungen besser wird und die Mutagenität deutlich verringert wird.

Es ist daher bevorzugt wenn $R^a$ bei jedem Auftreten gleich oder verschieden H, D oder eine Alkylgruppe mit 1 bis 20 C-Atomen oder ein aromatisches Ringsystem mit 6 bis 60 C-Ring-Atomen ist, in dem auch H-Atome durch D oder F ersetzt sein können; dabei können zwei oder mehrere benachbarte Substituenten $R^a$ auch miteinander ein mono- oder polycyclisches, aliphatisches, aromatisches oder heteroaromatisches Ringsystem bilden wobei bevorzugt ist, wenn zwei oder mehrere benachbarte Reste $R^a$ keinen Ringschluss bilden;

Aus dem selben Grund ist daher auch bevorzugt, wenn $R^b$ bei jedem Auftreten gleich oder verschieden eine Alkylgruppe mit 1 bis 20 C-Atomen, ein aromatisches Ringsystem mit 6 bis 60 C-Ring-Atomen ist, wobei H-Atome durch D oder F ersetzt sein können; dabei können zwei oder mehrere benachbarte Substituenten $R^b$ auch miteinander ein mono- oder polycyclisches, aliphatisches oder aromatisches Ringsystem bilden, wobei bevorzugt ist, wenn zwei oder mehrere benachbarte Reste $R^b$ keinen Ringschluss bilden

Weiterhin bevorzugt ist, wenn $R^a$ bei jedem Auftreten gleich oder verschieden H, D oder eine Alkylgruppe mit 1 bis 20 C-Atomen ist, wobei zwei oder mehrere benachbarte Reste $R^a$ keinen Ringschluss bilden.

Besonders bevorzugte Reste R sind die Gruppen der folgenden Formeln, wobei die gestrichelten Linien die Bindung zu dem Chinolin der Formel (1) kennzeichnen

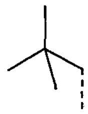

(R-1)

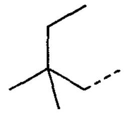

(R-2)

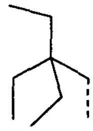

(R-3)

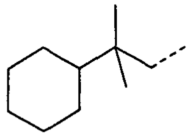

(R-4)

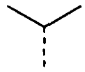

(R-5)

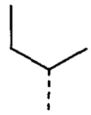

(R-6)

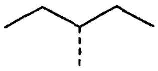

(R-7)

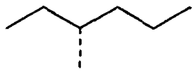

(R-8)

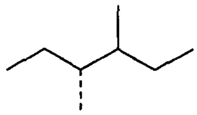

(R-9)

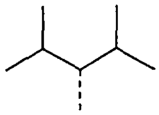

(R-10)

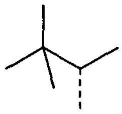

(R-11)

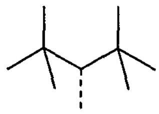

(R-12)

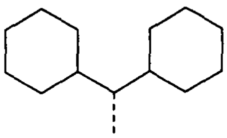

(R-13)

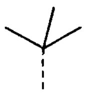

(R-14)

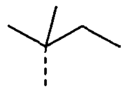

(R-15)

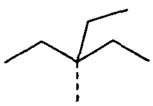

(R-16)

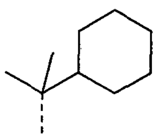

(R-17)

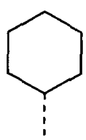

(R-18)

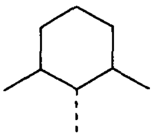
(R-19)

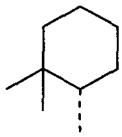
(R-20)

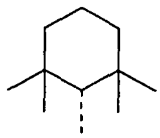
(R-21)

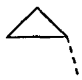
(R-22)

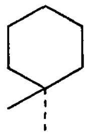
(R-23)

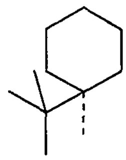
(R-24)

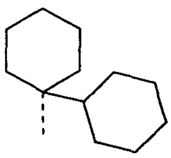
(R-25)

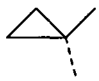
(R-26)

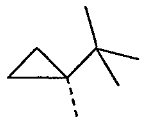
(R-27)

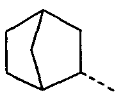
(R-28)

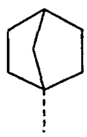
(R-29)

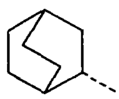
(R-30)

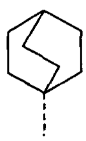
(R-31)

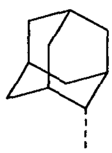
(R-32)

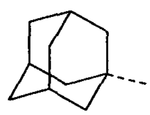
(R-33)

Ganz bevorzugt ist, wenn R über ein quartäres Kohlenstoffatom an den Chinolinring gebunden ist.

Es ist bevorzugt in Sinne der vorliegenden Erfindung, dass der Rest $R^a$ gleich H ist.

Besonders bevorzugt im Sinne der vorliegenden Erfindung ist eine Verbindung der folgenden Formel, wobei obige Definitionen und die in der vorliegenden Erfindung angegebenen bevorzugten Ausführungsformen für R, M, X und n auch bevorzugte Ausführungsformen für die Verbindung der Formel (A-1) darstellen und wobei die Verbindung noch mit einem oder mehreren, gleichen oder verschiedenen Resten $R^a$ substituiert sein kann, wobei ganz besonders bevorzugt ist, wenn $R^a$ in der Verbindung der Formel (A-1) gleich H ist

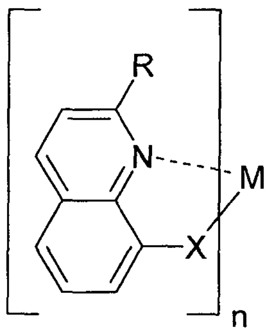

Formel (A-1)

Ganz besonders bevorzugt ist demnach auch eine Verbindung der Formel (A-1), wobei R eine verzweigte Alkylgruppe mit 4 bis 40 C-Atomen, bevorzugt mit 4 bis 30 C-Atomen, ganz bevorzugt mit 4 bis 20 C-Atomen, ganz besonders bevorzugt mit 4 bis 10 C-Atomen und insbesondere bevorzugt mit 4 bis 6 C-Atomen ist, wobei die Gruppe nicht weiter substituiert ist und wobei bevorzugt keiner der Reste R oder $R^a$ mit dem Chinolinring ein Ringsystem bildet und wobei noch mehr bevorzugt ist, wenn M gleich Li, X gleich O und n gleich 1 ist.

Bei der Verbindung der Formel (A-2) handelt es sich um eine ganz bevorzugte, wobei die Verbindung noch mit einem oder mehreren, gleichen oder verschiedenen Resten $R^a$ substituiert sein kann, wobei ganz besonders bevorzugt ist, wenn $R^a$ in der Verbindung der Formel (A-2) gleich H ist. Hierbei ist eine Verbindung der Formel (A-1) mit M gleich Li, n gleich 1 und X gleich O noch bevorzugter.

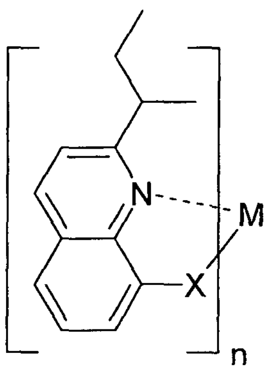

Formel (A-2)

Bei der Verbindung der Formel (A-3) handelt es sich um eine weitere ganz bevorzugte, wobei die Verbindung noch mit einem oder mehreren, gleichen oder verschiedenen Resten $R^a$ substituiert sein kann, wobei ganz besonders bevorzugt ist, wenn $R^a$ in der Verbindung der Formel (A-3) gleich H ist. Hierbei ist eine Verbindung der Formel (A-3) mit M gleich Li, n gleich 1 und X gleich O noch bevorzugter.

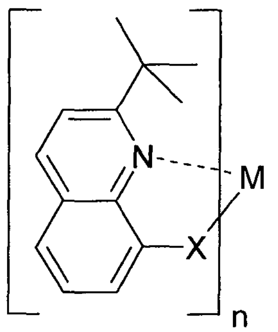

Formel (A-3)

Bei der Verbindung der Formel (A-4) handelt es sich um eine weitere ganz bevorzugte, wobei die Verbindung noch mit einem oder mehreren, gleichen oder verschiedenen Resten $R^a$ substituiert sein kann, wobei ganz besonders bevorzugt ist, wenn $R^a$ in der Verbindung der Formel (A-4) gleich H ist. Hierbei ist eine Verbindung der Formel (A-4) mit M gleich Li, n gleich 1 und X gleich O noch bevorzugter.

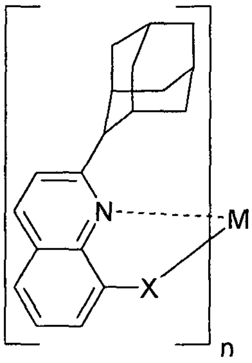

Formel (A-4)

Es ist weiterhin bevorzugt, wenn die Verbinndung der Formel (1) keine kondensierten aromatische oder kondensierten heteroaromatischen Ringsysteme mit mehr als 10 Ringatome enthält, und ganz bevorzugt ist, wenn sie überhaupt keine kondensierten aromatische oder kondensierten heteroaromatischen Ringsysteme enthält.

Weiterhin bevorzugt ist, wenn $R^b$ bei jedem Auftreten gleich oder verschieden eine Alkylgruppe mit 1 bis 20 C-Atomen ist, wobei bevorzugt ist wenn zwei oder mehrere benachbarte Reste $R^b$ keinen Ringschluss bilden.

Ganz bevorzugte Verbindungen der Formel (1) sind auch solche der Formeln (B-1) bis (B-9), wobei die Verbindungen noch mit einem oder mehreren, gleichen oder verschiedenen Resten $R^a$ substituiert sein können, wobei ganz besonders bevorzugt ist, wenn $R^a$ gleich H ist.

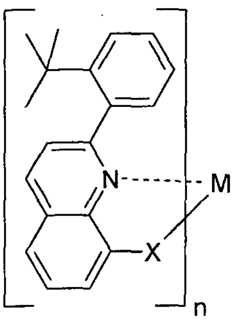

Formel (B-1)

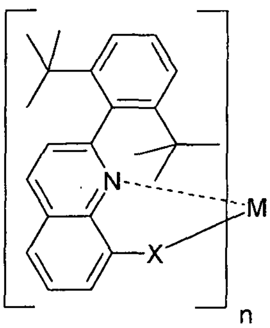

Formel (B-2)

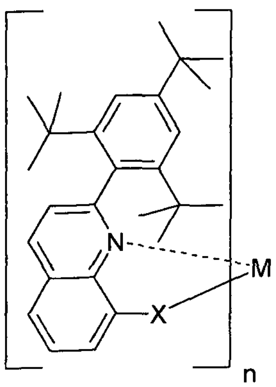

Formel (B-2)

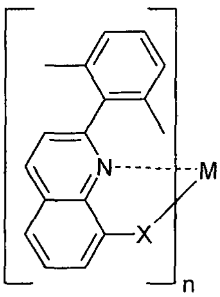

Formel (B-3)

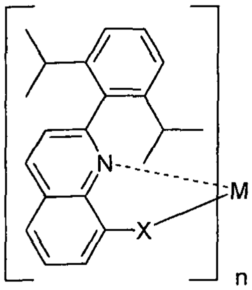

Formel (B-4)

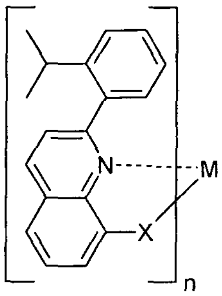

Formel (B-5)

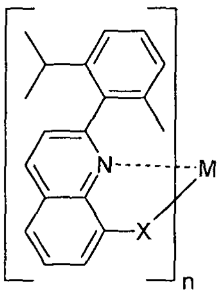

Formel (B-6)

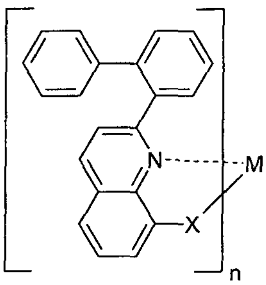

Formel (B-7)

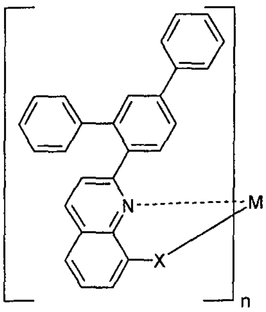

Formel (B-8)

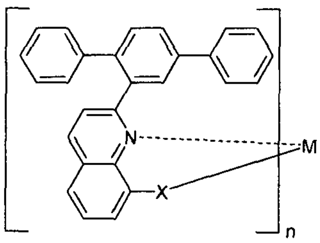

Formel (B-9)

Hierbei ist eine Verbindung der Formel (B-1) bis (B-9) mit M gleich Li, n gleich 1 und X gleich O noch bevorzugter Weitere Beispiele für erfindungsgemäße Verbindungen mit M gleich Li und n gleich 1 sind die im Folgenden abgebildeten Verbindungen der Formeln (C-1) bis (C-34).

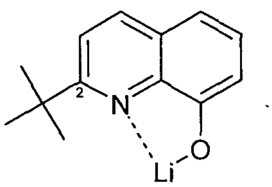

Formel (C-1)

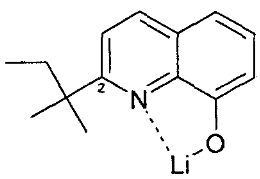

Formel (C-2)

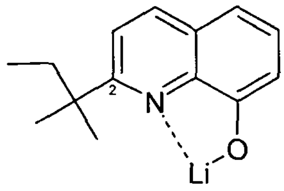

Formel (C-3)

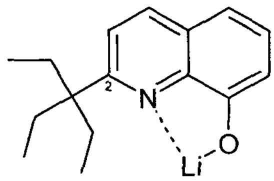

Formel (C-4)

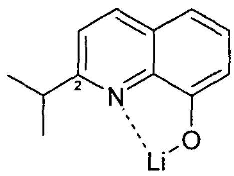

Formel (C-5)

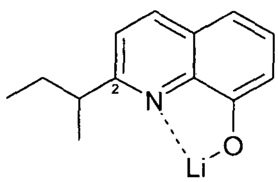

Formel (C-6)

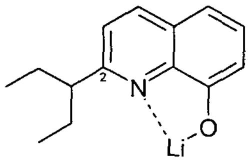

Formel (C-7)

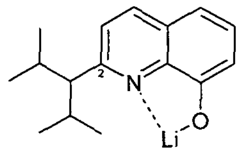

Formel (C-8)

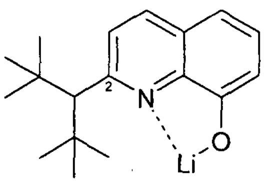

Formel (C-9)

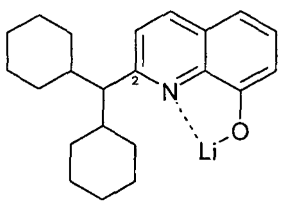

Formel (C-10)

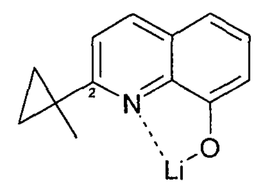

Formel (C-11)

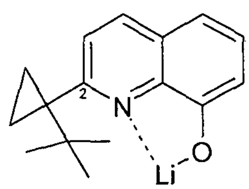

Formel (C-12)

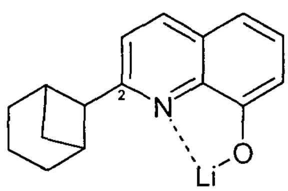

Formel (C-13)

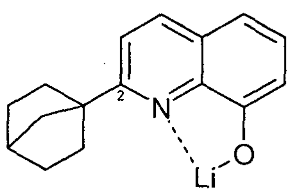

Formel (C-14)

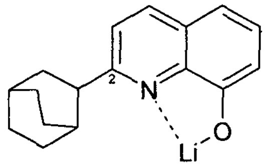

Formel (C-15)

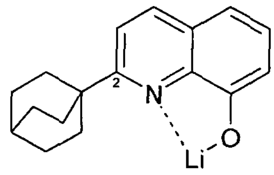

Formel (C-16)

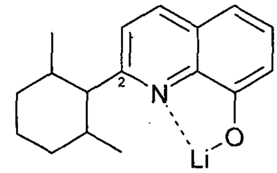

Formel (C-17)

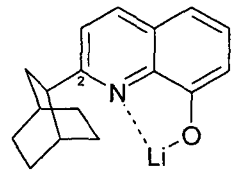

Formel (C-18)

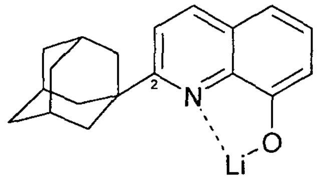

Formel (C-19)

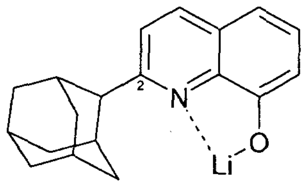

Formel (C-20)

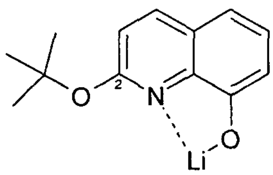

Formel (C-21)

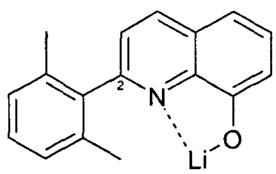

Formel (C-22)

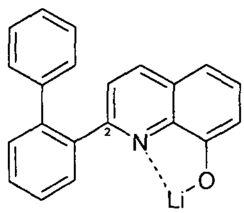

Formel (C-23)

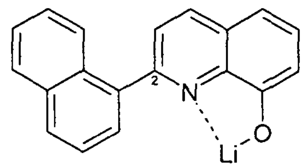

Formel (C-24)

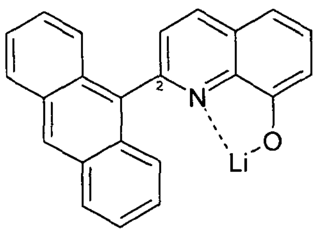

Formel (C-25)

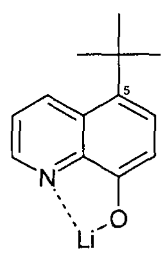

Formel (C-26)

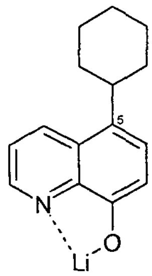

Formel (C-27)

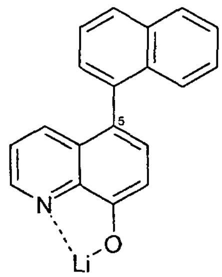

Formel (C-28)

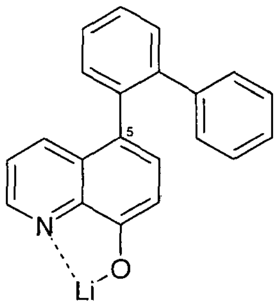

Formel (C-29)

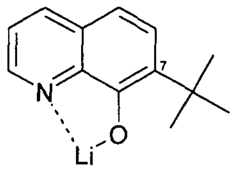

Formel (C-30)

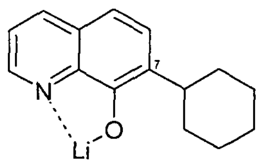

Formel (C-31)

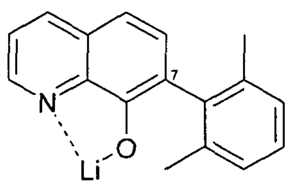

Formel (C-32)

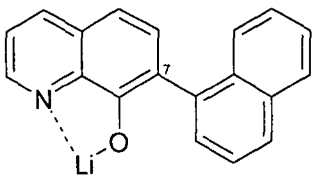

Formel (C-33)

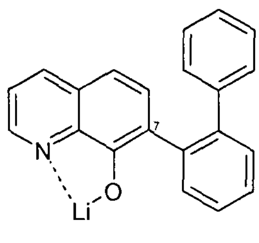

Formel (C-34)

Weitere Verbesserungen von Effizienzdaten von Vorrichtungen enthaltend die Verbindungen können erreicht werden, wenn die erfindungsgemäßen Verbindungen in Zusammensetzungen mit anderen Materialen eingesetzt werden. Bevorzugt werden die erfindungsgemäßen Verbindungen in Zusammensetzung mit Materialien eingesetzt, die typischerweise in Vorrichtungen, insbesondere elektronischen Vorrichtungen wie Elektrolumineszenzvorrichtungen, eingesetzt werden.

Die vorliegende Erfindung betrifft daher auch eine Zusammensetzung enthaltend eine oder mehrere erfindungsgemäße Verbindungen sowie mindestens ein zusätzliches funktionelles Molekül ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien, Lochblockiermaterialien und n-Dotanden.

Dem Fachmann sind die einzelnen Materialien gut bekannt und es bereitet ihm keinerlei Schwierigkeiten aus einem großen zur Verfügung stehenden Repertoire geeignete Verbindungen auszuwählen.

In einer weiteren bevorzugten Ausführungsform der Erfindung werden die Verbindungen gemäß Formel (1) als eine Komponente von Mixed-Matrix-Systemen verwendet. Die Mixed-Matrix-Systeme umfassen bevorzugt zwei oder

drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Die vorliegende Erfindung betrifft daher auch eine Zusammensetzung enthaltend wenigstens eine Verbindung gemäß Formel (1) sowie wenigstens ein weiteres Matrixmaterial.

Die vorliegende Erfindung betrifft auch eine Zusammensetzung enthaltend wenigstens eine Verbindung gemäß Formel (1) sowie wenigstens ein wide band gap Material, wobei unter wide band gap Material ein Material im Sinne der Offenbarung von US 7,294,849 verstanden wird. Diese Systeme zeigen besondere vorteilhafte Leistungsdaten in elektrolumineszierenden Vorrichtungen.

Systeme umfassend mehrere Matrixmaterialien (Mixed-Matrix-Systeme) werden insbesondere in lichtemittierenden Schichten organischer Elektrolumineszenzvorrichtungen eingesetzt. Die lichtemittierende Schicht enthält weiter noch einen oder mehrere Dotanden.

In einer bevorzugten Ausführungsform der vorliegenden Erfindung enthält die Zusammensetzung neben wenigstens einer Verbindung der Formel (1) wenigstens ein zusätzliches funktionelles Material, das eine Elektronentransportverbindung ist, wobei ganz bevorzugt ist, wenn das zusätzliche funktionelle Material ein nicht-metallisches Elektronentransportmaterial ist.

Bevorzugte zusätzliche Elektronentransportmaterialien, die in Zusammensetzung mit den erfindungsgemäßen Verbindungen eingesetzte werden sind Pyridine, Pyrimidine, Pyridazine, Pyrazine, Oxadiazole, Oxazole, Lactame, Chinoline, Chinoxaline, Anthracene, Benzanthracene, Pyrene, Perylene, Benzimidazole, Triazine, Ketone, Lactame, Phosphinoxide und Phenazine und ganz bevorzugt Triazine.

Eine bevorzugte Zusammensetzung im Sinne der vorliegenden Erfindung enthält wenigstens eine Verbindung der allgemeinen Formel (1) sowie ein 8-Hydroxychinolinat, das nicht unter den Schutzumfang der vorliegenden Erfindung fällt. Insbesondere zählen hierzu $Hfq_4$, $Zrq_4$, $Alq_3$ und Liq, wobei q für den Liganden 8-Hydroxychinolinat steht. Besonders bevorzugt ist dabei Liq. Idealerweise liegt die Verbindung der Formel (1) in einer höheren Konzentration in der Zusammensetzung vor, als die zweite Chinolinverbindung. Das hat den technischen Vorteil, dass die Leistungsdaten elektronischer Vorrichtungen, insbesondere von organischen Elektrolumineszenzvorrichtungen, weiter verbessert werden, aber die mutagenen Verbindungen in niedrigeren Konzentrationen eingesetzt werden können. Vorrichtungen enthaltend diese Zusammensetzungen sind folglich weniger gesundheitsgefährdend.

Ganz bevorzugt betrifft die vorliegende Erfindung Zusammensetzungen enthaltend mindestens eine Verbindung gemäß Formel (1) sowie mindestens eine Verbindung der folgenden Formel (2)

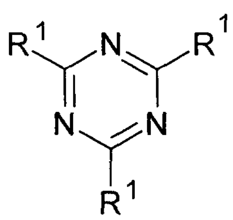

Formel (2)

wobei für die verwendeten Symbole und Indizes gilt:

$R^1$ ist bei jedem Auftreten gleich oder verschieden H, D, F, Cl, Br, I, CHO, $N(R^2)_2$, $N(Ar^1)_2$, $B(Ar^1)_2$, $C(=O)Ar^1$, $P(=O)(Ar^1)_2$, $S(=O)Ar^1$, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, eine geradkettige Alkyl-, Alkenyl-, Alkinyl-, Alkoxy- oder Thioalkoxygruppe mit 1 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit

einem oder mehreren Resten $R^2$ substituiert sein kann, wobei eine oder mehrere nicht benachbarte $CH_2$-Gruppen durch $R^2C=CR^2$, C=C , $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden;

$Ar^1$ ist bei jedem Auftreten gleich oder verschieden ein aromatisches oder heteroaromatisches Ringsystem mit 5 bis 30 aromatischen Ring-atomen, das mit einem oder mehreren Resten $R^2$ substituiert sein kann; dabei können auch zwei Reste $Ar^1$, welche an dasselbe Stickstoff-, Phosphor- oder Boratom binden, durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verknüpft sein;

$R^2$ ist bei jedem Auftreten gleich oder verschieden H, D oder ein aliphatischer, aromatischer und/oder heteroaromatischer Kohlenwasserstoffrest mit 1 bis 20 C-Atomen, in dem auch H-Atome durch D oder F ersetzt sein können; dabei können zwei oder mehrere benachbarte Substituenten $R^2$ auch miteinander ein mono- oder polycyclisches, aliphatisches oder aromatisches Ringsystem bilden.

Bevorzugt weisen die zusätzlichen Elektronentransportmaterialien eine Glasübergangstemperatur $T_G$ von größer als 70°C auf, besonders bevorzugt größer als 90°C, ganz besonders bevorzugt größer als 110°C, bestimmt nach DIN 51005.

Bevorzugt sind hierbei Triazine der Formel (2), bei denen mindestens einer der Reste $R^1$, vorzugsweise mindestens zwei der Reste $R^1$ und besonders bevorzugt alle Reste $R^1$ unabhängig voneinander ein aromatisches oder heteroaromatisches Ringsystem mit 5 bis 60 aromatischen Ringatomen bedeuten.

Ferner kann vorgesehen sein, dass die Verbindung gemäß Formel (2) ein Carbazolderivat, vorzugsweise ein Indeno- oder ein Indolo- Carbazolderivat, oder ein Biphenylderivat, vorzugsweise ein Terphenyl- oder ein Quaterphenylderivat ist.

Weiter bevorzugte zusätzliche Elektronentransportmaterialien sind Triazine, die in WO 2010/072300 A1 offenbart werden. Die genannten Triazin-Verbindungen können beispielsweise gemäß den in US 6,229,012, US 6,225,467, WO 05/053055 und DE 102008036982.9 beschriebenen Verfahren synthetisiert werden.

In einer weiteren bevorzugten Ausführungsform der vorliegenden Erfindung enthält die Zusammensetzung neben wenigstens einer Verbindung der Formel (1) wenigstens ein zusätzliches funktionelles Material, das ein n-Dotand ist. Der n-Dotand kann dabei sowohl ein anorganisches wie auch ein organisches Material sein.

Bei einer n-Dotierung erfolgt ein Elektronentransfer vom HOMO (highest occupied molecular orbital) Niveau des n-Dotanden zum LUMO (lowest unoccupied molecular orbital) Niveau des Matrixmaterials, wobei das Elektron im Allgemeinen nicht stark lokalisiert ist, sondern zu den Ladungsträgern beizählt.

Eine Weiterbildung der Erfindung sieht vor, dass der Betrag einer Differenz zwischen dem HOMO des n-Dotanden und dem LUMO der erfindungsgemäßen Verbindung bevorzugt kleiner als etwa 1 eV ist, weiter bevorzugt ist der Betrag der Differenz kleiner als etwa 0.5 eV.

Bevorzugt haben die erfindungsgemäßen Verbindungen ein LUMO Niveau von etwa 1 eV oder größer, ganz bevorzugt von 1.5 eV oder größer.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden vorliegend mit Hilfe quantenchemischer Rechnungen bestimmt. Zur Berechnung organischer Substanzen ohne Metalle wird zuerst eine Geometrieoptimierung mit der Methode "Ground State/Semiempirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die zur n-Dotierung eingesetzten Verbindungen können als Precursor eingesetzt werden, wobei diese Precursor-Verbindungen durch Aktivierung n-Dotanden freisetzen.

Bevorzugten sind n-Dotanden ausgewählt aus elektronenreichen Metallkomplexen; P=N-Verbindungen; N-Heterocyclen, besonders bevorzugt, Naphthylencarbodiimide, Pyridinen, Acridinen und Phenazinen; Fluorenen und Radikal-Verbindungen.

Besonders bevorzugte elektronenreichen Metallkomplexe werden unter anderem in WO 2005/86251 A2 beschrieben, wobei diese Druckschrift zu Offenbarungszwecken in die vorliegende Anmeldung durch Referenz hierauf eingefügt wird. Hierbei sind neutrale elektronenreiche Metallkomplexe bevorzugt.

Besonders bevorzugte P=N-Verbindungen werden unter anderem in WO 2012/175535 A1 offenbart, wobei diese Druckschrift zu Offenbarungszwecken in die vorliegende Anmeldung durch Referenz hierauf eingefügt wird.

Eine weitere Gruppe von n-Dotanden stellen N-Heterocyclen dar. N-Heterocyclen sind cyclische Verbindungen, deren Ringstruktur neben Wasserstoff und Kohlenstoff mindestens ein Stickstoffatom aufweist. Diese Verbindungen können gesättigt, teilweise ungesättigt oder heteroaromatisch sein.

N-Heterocyclen können bevorzugt als Precursor eingesetzt werden, wobei sich Precursor-Verbindungen dadurch auszeichnen, dass ihre Wirkung als n-Dotand erst nach einer Aktivierung einsetzt. Bevorzugte N-Heterocyclen, die sich insbesondere als Precursor eingesetzt werden können, sind unter anderem in WO 2009/00237 A1 beschrieben, wobei diese Druckschrift zu Offenbarungszwecken in die vorliegende Anmeldung durch Referenz hierauf eingefügt wird.

Eine weitere Gruppe N-Heterocyclen, die sich als n-Dotand eignen stellen Naphthylencarbodiimide dar. Naphthylencarbodiimide umfassen mindestens eine Carbodiimid-Gruppe (N=C=N) und eine Naphthylen-Gruppe.

Überraschende Vorteile können durch die in WO 2012/168358 A1 beschriebenen Naphthylencarbodiimide erzielt werden, wobei diese Druckschrift zu Offenbarungszwecken in die vorliegende Anmeldung durch Referenz hierauf eingefügt wird.

Zu den bevorzugten, als n-Dotanden einsetzbare N-Heterocylcen zählen weiterhin Pyridin-, Acridin- und Phenazin-Derivate. Diese Verbindungen umfassen Pyridin-, Acridin- und Phenazin-Strukturelemente und sind in der Fachwelt bekannt. Bevorzugte Acridine und Phenazine sind unter anderem in US 2007/0145355 A1 dargelegt, wobei diese Druckschrift zu Offenbarungszwecken in die vorliegende Anmeldung durch Referenz hierauf eingefügt wird.

Überraschende Vorteile können durch die in EP 2 452 946 A1 und EP 2 463 927 A1 beschriebenen Pyridine erzielt werden, wobei diese Druckschriften zu Offenbarungszwecken in die vorliegende Anmeldung durch Referenz hierauf eingefügt wird.

Gemäß einer besonderen Ausgestaltung der vorliegenden Erfindung können Fluorene als n-Dotanden eingesetzt werden.

Bevorzugte Fluorene sind unter anderem in WO 2012/031735 A1 beschrieben, wobei diese Druckschrift zu Offenbarungszwecken in die vorliegende Anmeldung durch Referenz hierauf eingefügt wird.

Zu den bevorzugten n-Dotanden zählen Radikal-Verbindungen, die in der Fachwelt bekannt sind. Bevorzugte Radikal-Verbindungen umfassen heterocyclische Gruppen. Besonders bevorzugte Radikal-Verbindungen sind unter anderem in EP 1 837 926 A1 und WO 2007/107306 A1 offenbart, wobei diese Druckschrift zu Offenbarungszwecken in die vorliegende Anmeldung durch Referenz hierauf eingefügt wird.

Von den genannten n-Dotanden sind die in WO 2005/86251 A2 dargelegten elektronenreichen Metallkomplexe besonders bevorzugt, wobei die Metallkomplexe der Formel $W_2(hpp)_4$ ganz besonders bevorzugt sind, worin hpp für das Anion von 1, 3, 4, 6, 7, 8-Hexahydro-2H-pyrimido [1, 2-a] pyrimidin steht. Hierbei sind neutrale elektronenreiche Metallkomplexe besonders bevorzugt.

Gegenstand der vorliegenden Erfindung sind auch Formulierungen enthaltend wenigstens eine der erfindungsgemäßen Verbindungen oder wenigstens eine der erfindungsgemäßen Zusammensetzungen sowie wenigstens ein Lösungsmittel.

Besonders vorteilhaft sind solche Formulierungen, um Vorrichtungen aus Lösung, d.h. aus flüssiger Phase mittels Drucktechnologien zu prozessieren. Für die Verarbeitung der erfindungsgemäßen Verbindungen und Zusammensetzungen aus flüssiger Phase, beispielsweise durch SpinCoating oder durch Druckverfahren, sind Formulierungen der erfindungsgemäßen Verbindungen und Zusammensetzungen erforderlich. Diese Formulierungen können beispielsweise Lösungen, Dispersionen oder Emulsionen sein. Es kann bevorzugt sein, hierfür Mischungen aus zwei oder mehr Lösungsmitteln zu verwenden. Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethyheneglycolbutylmethyl ether, Triethyleneglycolbutylmethyl ether, Diethyleneglycoldibutyl ether, Triethylenglycoldimethyl ether, Diethylenglycolmonobutylether, Tripropylenglycol dimethyl ether, Tetraethyleneglycol dimethyl ether, 2-isopropyl naphthalin, Pentylbenzol, Hexylbenzol, Heptylbenzol, octylbenzol, 1,1-Bis(3,4-Dimethylphenyl)ethan oder Mischungen dieser Lösungsmittel.

Die erfindungsgemäßen Verbindungen und Zusammensetzungen können in Vorrichtungen, bevorzugt in elektronischen Vorrichtungen verwendet werden. Ganz bevorzugt werden die erfindungsgemäßen Verbindungen und Zusammensetzungen in einer organischen Elektromunieszenzvorrichtung, ganz besonders bevorzugt in einer organischen lichtemittierenden Diode (OLED) oder in einer organischen lichtemittierenden elektrochemischen Zelle (OLEC, LEC oder auch LEEC) und insbesondere bevorzugt in einer OLED verwendet.

Zu den OLEDs werden vorliegend auch die polymeren lichtemittierenden Dioden PLEDs gezählt.

Die vorliegende Erfindung betrifft auch eine Vorrichtung enthaltend mindestens eine der erfindungsgemäßen Verbindungen oder mindestens eine der erfindungsgemäßen Zusammensetzungen

Bevorzugt handelt es sich bei den erfindungsgemäßen Vorrichtungen um eine elektronische Vorrichtung.

Ganz bevorzugte Vorrichtungen im Sinne der vorliegenden Erfindung sind die organischen Elektrolumineszenzvorrichtungen, den organischen integrierten Schaltungen; organischen Feld-Effekt-Transistoren, organischen Dünnfilmtransistoren; organischen Solarzellen; organischen optischen Detektoren; organischen Photorezeptoren oder organischen Feld-Quench-Devices, wobei die organischen Elektrolumineszenzvorrichtungen ganz besonders bevorzugt sind.

Insbesondere bevorzugt sind organische Elektrolumineszenzvorrichtungen, die ausgewählt sind aus der Gruppe bestehend aus den organischen lichtemittierenden Dioden (OLED), den organischen lichtemittierenden Transistoren, den organischen lichtemittierenden elektrochemischen Zellen (OLEC, LEC oder auch LEEC) und den organischen Laserdioden, wobei hieraus die OLEDs und OLECs noch bevorzugter sind und wobei die OLEDS am meisten bevorzugt sind.

Eine erfindungsgemäße elektronische, insbesondere elektrolumineszierende, organische Vorrichtung umfasst mindestens eine organische Schicht, die mindestens eine Verbindung der Formel (1) enthält. Eine organische Schicht zeichnet sich dadurch aus, dass diese mindestens eine organische bzw. metallorganische Verbindung enthält. Eine organische Vorrichtung muss nicht notwendigerweise nur Schichten enthalten, welche aus organischen oder metallorganischen Materialien aufgebaut sind. So ist es auch möglich, dass eine oder mehrere Schichten anorganische Materialien enthalten oder ganz aus anorganischen Materialien aufgebaut sind. Vorzugsweise kann eine organische Schicht, insbesondere die organische Schicht, die mindestens eine Verbindung der Formel (1) enthält, mindestens 30 Vol.-%, ganz bevorzugt mindestens 60 Vol.-%, ganz besonders bevorzugt mindestens 90 Vol.%, und insbesondere bevorzugt 100 Vol.-% an organischen oder metallorganischen Materialien umfassen.

Bevorzugt beträgt der Anteil der Verbindung gemäß Formel (1) in der Schicht mindestens 5 Vol.-%, ganz bevorzugt mindestens 15 Vol.-%, ganz besonders bevorzugt mindestens 40 Vol.-%,insbesondere bevorzugt mindestens 60 Vol.-%, noch bevorzugter mindestens 80 Vol.-% und am meisten bevorzugt mindestens 90 Vol.-%. In einer ganz bevorzugten Ausführungsform besteht die Schicht vollständig aus einer Verbindung der Formel (1).

Verbindungen der Formel (1) können in der mindestens einen organischen Schicht als Reinsubstanz oder als Mischung von zwei oder mehr Verbindungen der Formel (1) eingesetzt werden. Vorzugsweise kann die organische Schicht zwei Verbindungen der Formel (1) umfassen, die in Form einer Mischung vorliegen, wobei sich die mindestens zwei Verbindungen der Formel (1) vorzugsweise durch die Position unterscheiden an der R an den Chinolinring gebunden ist. Besonders bevorzugt sind hierbei Mischungen, bei denen mindestens eine Verbindung der Formel (1) enthalten ist, bei dem der Rest R an Position 2 an den Chinolinring gebunden ist. Weiterhin sind Mischungen von besonderem Interesse, enthaltend mindestens eine Verbindungen der Formel (1), bei dem der Rest R an Position 5 oder 7 an den Chinolinring gebunden ist. Das Volumenverhältnis der mindestens zwei Verbindungen der Formel (1) in der organischen Schicht kann vorzugsweise im Bereich von 10:1 bis 1:10, besonders bevorzugt im Bereich von 5:1 bis 1:5 und speziell

bevorzugt im Bereich von 2:1 bis 1:2 liegen.

Die erfindungsgemäßen Verbindungen und Zusammensetzungen weisen elektronenleitende Eigenschaften auf und befinden sich vorzugsweise in einer elektronenleitenden Schicht der Vorrichtung.

Die erfindungsgemäßen Verbindungen und Zusammensetzungen befinden sich vorzugsweise in einer Elektronentransportschicht der Vorrichtung.

Die erfindungsgemäßen Verbindungen und Zusammensetzungen befinden sich vorzugsweise in einer Elektroneninjektionsschicht der Vorrichtung

Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine Verbindung der Formel (1) umfasst. Dabei sind bevorzugte elektronische Vorrichtungen ausgewählt aus der Gruppe bestehend aus organischen Elektrolumineszenzvorrichtungen, vorzugsweise organischen lichtemittierenden Dioden (OLED, PLED), organischen lichtemittierenden Transistoren (O-LETs), lichtemittierenden elektrochemischen Zellen (OLEC) oder organischen Laser-dioden; organischen integrierten Schaltungen (O-ICs), organischen Feld-Effekt-Transistoren (O-FETs), organischen Dünnfilmtransistoren (O-TFTs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser).

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat), wobei erfindungsgemäße Li-Hydroxychinolin der Formel (1) besonders bevorzugt sind. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. $Al/Ni/NiO_x$, $Al/PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $MoO_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

Gemäß einer bevorzugten Ausführungsform können zwischen der Anode und der Katode mindestens zwei organische Schichten angeordnet sein, wobei in mindestens zwei organischen Schichten mindestens eine Verbindung der Formel (1) enthalten ist.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Besonders bevorzugt sind organische Elektrolumineszenzvorrichtungen. Eine organische Elektrolumineszenzvorrichtung enthält Kathode, Anode und mindestens eine emittierende Schicht (EML). Außer diesen Schichten kann sie noch weitere Schichten enthalten, beispielsweise jeweils eine oder mehrere Lochinjektionsschichten (HIL), Lochtransportschichten (HTL), Lochblockierschichten (HBL), Elektronentransportschichten (ETL), Elektroneninjektionsschichten (EIL), Exzitonenblockierschichten (ExBL), Elektronenblockierschichten (EBL), Ladungserzeugungsschichten und/oder organische oder anorganische p/n-Übergänge. Ein typischer Aufbau einer organischen Elektrolumineszenzvorrichtung ist:

Anode/HIL/HTL/EML/ETL/EIL/Kathode

Dabei ist es möglich, dass eine oder mehrere Lochtransportschichten p-dotiert sind, beispielsweise mit Metall-

oxiden, wie $MoO_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

Vorzugsweise kann die mindestens eine Verbindung gemäß Formel (1) enthaltende organische Schicht mit elektronenleitenden Eigenschaften eine Elektronentransportschicht oder eine Elektroneninjektionsschicht sein.

Als besonders vorteilhaft hat sich weiterhin eine organische Elektrolumineszenzvorrichtung erwiesen, enthaltend Anode, Kathode, mindestens eine emittierende Schicht und mindestens eine Elektronentransportschicht, welche zwischen der emittierenden Schicht und der Kathode angeordnet ist. Besonders bevorzugt umfasst die zwischen der emittierenden Schicht und der Kathode angeordnete Elektronentransportschicht mindestens eine Verbindung der Formel (1).

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend mindestens einen Emitter und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Schwierigkeiten auf elektronischen Vorrichtungen, insbesondere organische Elektrolumineszenzvorrichtungen enthaltend eine Verbindung der Formel (1) oder die oben aufgeführten bevorzugten Ausführungsformen angewendet werden.

Die vorliegende Erfindung betrifft auch Verfahren zur Herstellung der erfindungsgemäßen Verbindungen.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

So können die zuvor dargelegten Verbindungen der Formel (1) aus 8-Hydroxychinolin über Lithium-Verbindungen, Boron-Minisci-Reaktionen und Zink-Sulfinate erhalten werden.

Die Herstellung von Verbindungen der Formel (1) über Lithium-Verbindungen ausgehend von 8-Hydroxychinolin gemäß dem allgemeinen Schema

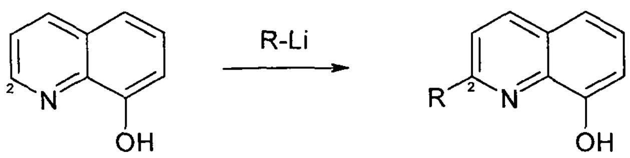


ist unter anderem in Tetrahedron Asymmetry, 12 (2001), 1345 dargestellt, wobei hierauf Bezug genommen wird, wobei der Rest R die zuvor definierte Bedeutung hat.

Weiterhin sind die erfindungsgemäßen Verbindungen der Formel (1) über eine Boron-Minisci-Reaktion gemäß

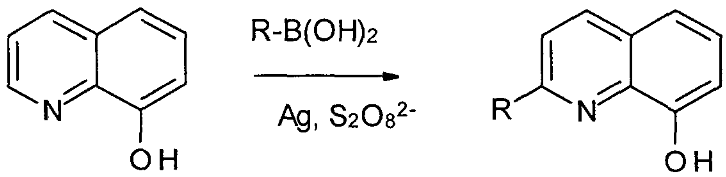


erhältlich, wobei der Rest R die zuvor definierte Bedeutung hat. Weitere Informationen bezüglich bevorzugten Ausführungsformen einer Boron-Minisci-Reaktion sind in J. Am. Chem. Soc., 2010, 132, 13194 dargestellt.

Über Zink-Sulfinate können ebenfalls zur Herstellung von erfindungsgemäßen Verbindungen der Formel (1) gemäß dem Schema

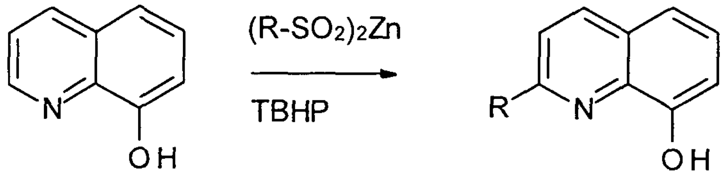


dienen, wobei der Rest R zuvor definierte Bedeutung hat. Bevorzugte Ausführungsformen dieser Herstellungsmethode sind unter anderem in Nature, 2012, 492, 95 beschrieben.

Weiterhin kann eine Bromierung von 8-Hydroxychinolin erfolgen, wobei das bromierte 8-Hydroxychinolin nachfolgend in einer Suzuki- oder Nigishi-Reaktion umgesetzt wird. Dieser Reaktionsweg wird nachfolgend schematisch dargestellt:

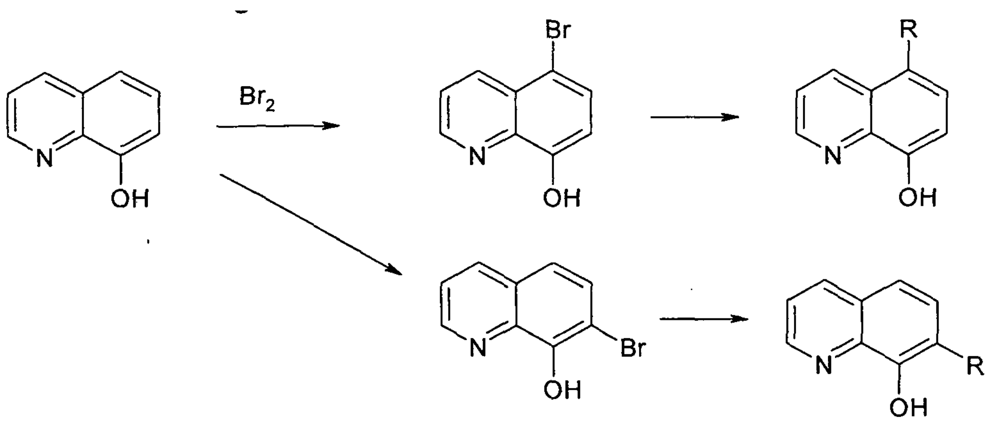


Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (1) und/oder Formel (II) in hoher Reinheit, bevorzugt mehr als 99 % (bestimmt mittels $^{1}$H-NMR und/oder HPLC) erhalten.

Die entsprechenden Verbindungen der Formel (1) mit X gleich S können analog hergestellt werden.

In einem letzten Schritt werden die Hydroxy- bzw. Thiolverbindungen mit Butyl-Lithium (BuLi) in Hexan umgesetzt, um den Metallkomplex gemäß Formel (1) zu erhalten. Als Lösungsmittel kann hierfür Acetonitril verwendet werden. Einzelheiten der Verfahrens sind in den Beispielen beschrieben.

Vorrichtungen enthaltend die Verbindungen nach Formel (1) können sehr vielseitig eingesetzt werden. So können, beispielsweise, elektrolumineszierende Vorrichtuungen enthaltend eine oder mehrere Verbindungen nach Formel (1) in Displays für Fernseher, Mobiltelefone, Computer und Kameras eingesetzt werden. Die Vorrichtungen können aber auch in Beleuchtungsanwendungen verwendet werden. Weiterhin können elektrolumineszierende Vorrichtungen, z. B. in OLEDs oder OLECs, enthaltend wenigstens eine der Verbindung nach Formel (1) in der Medizin oder Kosmetik zu Phototherapie genutzt werden. Somit kann eine Vielzahl von Erkrankungen (Psoriasis, atopische Dermatitis, Inflammation, Akne, Hautkrebs etc.) oder die Vermeidung sowie Reduktion von Hautfaltenbildung, Hautrötung und Hautalterung behandelt werden. Weiterhin können die lichtemittierenden Vorrichtungen dazu genutzt werden, um Getränke, Speisen oder Lebensmittel frisch zu halten oder um Geräte (bspw. medizinische Geräte) zu sterilisieren.

Gegenstand der vorliegenden Erfindung ist daher eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (1) zur Verwendung in der Medizin zur Phototherapie.

Ein weiterer bevorzugter Gegenstand der vorliegenden Erfindung betrifft eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (1) zur Verwendung zur phototherapeutischen Behandlung von Hautkrankheiten.

Ein weiterer ganz bevorzugter Gegenstand der vorliegenden Erfindung betrifft eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (1) zur Verwendung zur phototherapeutischen Behandlung von Psoriasis, atopische Dermatitis, Entzündungserkrankungen, Vitiligo, Wundheilung und Hautkrebs.

Die vorliegende Erfindung betrifft weiterhin die Verwendung der elektronischen Vorrichtung, bevorzugt einer organischen Elektrolumineszenzvorrichtung, ganz bevorzugt einer OLED oder OLEC und ganz besonders bevorzugt einer OLED enthaltend wenigstens eine Verbindung gemäß Formel (1) in der Kosmetik, bevorzugt zur Behandlung von Akne, alternder Haut (Skin Ageing), und von Zellulithe.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronischen Vorrichtungen, insbesondere organische Elektrolumineszenzvorrichtungen enthaltend eine Verbindung der Formel (1) als elektronenleitende Materialien weisen eine sehr gute Lebensdauer auf.

2. Elektronischen Vorrichtungen, insbesondere organische Elektrolumineszenzvorrichtungen enthaltend eine Verbindung der Formel (1) weisen eine hervorragende Effizienz auf.

3. Mit Verbindungen der Formel (1) kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

4. Die Verwendung von Verbindungen der Formel (1) in Schichten elektronischer Vorrichtungen, insbesondere organischer Elektrolumineszenzvorrichtungen führt zu einer hohen Mobilität der Elektron-Leiterstrukturen.

5. Verbindungen der Formel (1) zeichnen sich durch eine ausgezeichnete thermische Stabilität aus, wobei Verbindungen mit einer Molmasse von weniger als ca. 1200 g/mol gut sublimierbar sind.

6. Verbindungen der Formel (1) lassen sich aufgrund ihrer vorteilhaften Verdampfungsrate sehr gut prozessieren und eignen sich für die Massenproduktion von elektronischen Vorrichtungen.

7. Verbindungen der Formel (1) und Zusammensetzungen enthaltend diese zeigen eine geringe Toxizität und Mutagenität. Weiterhin sind diese Verbindungen und Zusammensetzungen sehr umweltverträglich. Die geringe Mutagenität kann insbesondere gemäß dem AMES-Test gezeigt werden.

8. Die erfindungsgemäßen Zusammensetzungen weisen verbesserte Leistungsdaten elektronischer Vorrichtungen auf.

Diese oben genannten Vorteile gehen nicht mit einer Verschlechterung der weiteren elektronischen Eigenschaften einher.

Die oben genannten bevorzugten Ausführungsformen sind beliebig miteinander kombinierbar. In einer besonders bevorzugten Ausführungsform der Erfindung gelten die oben genannten bevorzugten Ausführungsformen gleichzeitig.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

Abbildung 1 zeigt die Verdampfungskurven (p=p(T)) der erfindungsgemäßen Verbindungen E2-E5 und der Vergleichsverbindungen V1-V3. Die Kurven sind bei T=380°C von hohen Drücken zu niedrigen Drücken in der Reihenfolge E4, E3, E5, E2, V3, V1 und V2.

## Beispiele

### Beispiel 1

**Synthese von 2-tert-Butyl-8-hydroxychinolin**

60 g 8-Hydroxychinolin (0,41 mol) werden in 200 mL THF gelöst. Die Lösung wird auf -70°C gekühlt. Es werden 720 mL 1,7 M (3 eq.) tert Buthyllithium zugetropft. Die gelbe Suspension wird eine Stunde bei -70°C gerührt, und dann langsam auf Raumtemperatur erwärmt. Zu dieser Lösung werden dann 71 g (0,8 mol) tert-Butylhydroperoxid gegeben und über Nacht gerührt. Die Lösung wird mit 500 mL Toluol erweitert und einmal mit 1 N HCl und dreimal mit Wasser gewaschen. Das Lösungsmittel wird im Vakuum entfernt und der Rückstand wird über Kieselgel (Heptan:Essigester 10:1) chromatographiert. Das Material wird einmal aus Toluol/Heptan umkristallisiert. Man erhält 41,2 g (0,20 mol, 50%) 2-tert-Butyl-8-hydroxychinolin als farblosen Feststoff.

### Beispiel 2

**Synthese von Lithium 2-tert-Butyl-8-hydroxychinolin (E2)**

37 g (184 mmol) 2-tert-Butyl-8-hydroxychinolin aus Beispiel 1 werden in 250 mL Acetonitril gelöst. 108 mL 2,5 m n-BuLi in Hexan (270 mmol, 1,5 eq) werden bei 0°C zugetropft. Nach Rühren über Nacht wird der gebildete Feststoff abfiltiert, mit trockenem Acetonitril gewaschen und getrocknet. Man erhält 36 g (174 mmol, 95%) Lithium 2-tert-Butyl-8-hydroxychinolat als grauen Feststoff. Das Produkt wird zweimal im Hochvakuum (350°C/1 x $10^{-5}$ mbar) sublimiert. Man erhält 26 g (72%) sublimiertes Produkt

Analog dazu werden weitere erfindungsgemäße Verbindungen nach Formel (1) hergestellt.

### Beispiel 3

**Synthese von Lithium 2-(2,5-dimethylphenyl)-8-hydroxychinolin (E3)**

Die Synthese erfolgt analog zu der in J. Am. Chem. Soc, 2010, 132, 13194 offenbarten Methode.

36 g 8-Hydroxychinolin (0,25 mol) werden in 125 mL Dichlormethan gelöst und 19 ml (0,25 mol) Trifluoressigsäure wird hinzugefügt. Danach werden 54 g (0,37 mol) 2,5-Dimethylphenylboronsäure (Frontier Scientific) sowie 8,5 g (0,05 mol) Silbernitrat zugefügt. Es wird unter Eiskühlung eine Lösung von 100 g (0,38 mol) Kaliumperoxodisulfat in

1000 ml Wasser zugefügt und auf Raumtemperatur erwärmt. Die gelbe Suspension wird 24 Stunden gerührt. Es wird mit 1 Dichlormethan erweitert, die Phasen getrennt und die organische Phase wird zweimal mit Natriumhydrogencarbonat Lösung gewaschen. Das Lösungsmittel wird im Vakuum entfernt und der Rückstand wird über Kieselgel (Heptan:Essigester 10:1) chromatographiert. Das Material wird einmal aus Toluol/Acetonitril umkristallisiert. Man erhält 18,2 g (73 mmol, 29%) 2-(2,5-Dimethylphenyl)-8-hydroxychinolin als farblosen Feststoff.

10 g des Produktes werden analog zu Beispiel 2 zu 7,1 g Lithium-2-(2,5-dimethylphenyl)-8-hydroxychinolin umgesetzt, welches bei 330°C sublimiert wird.

## Beispiel 4

### Synthese von Lithium 2-(2,6-dimethylphenyl)-8-hydroxychinolin (E4)

Analog Beispiel 3 wird 2,6-Dimethylphenylboronsäure (Frontier Scientific) zu Lithium 2-(2,6-Dimethylphenyl)-8-hydroxychinolin umgesetzt und bei 325°C sublimiert.

## Beispiel 5

### Synthese von Lithium 4-(2,6-dimethylphenyl)-8-hydroxychinolin (E5)

10 g (44 mmol) 4-Brom-8 hydroxychinolin (Monatshefte für Chemie (1991), 122(11), 935-41) werden in 100 ml Toluol gelöst. Es werden 100 ml Wasser, 7,3 g (50 mmol) 2,6-Dimethylphenylboronsäure, 20,2 g (88 mmol) Kaliumphosphat-hydrat, 200 mg Palladiumacetat sowie 750 mg 2-Dicyclohaxylphosphino-2',6'dimethooxybiphenyl (= S-PHOS) zugegeben. Nach 12 Stunden Erhitzen zum Rückfluss wird abgekühlt, die Phasen getrennt, eingeengt und der zurückbleibende Feststoff wird über Kieselgel (Heptan:Essigester 10:1) chromatographiert. Das Material wird einmal aus Toluol/Acetonitril umkristallisiert. Man erhält 8.0 g (32 mmol) 4-(2,6-dimethylphenyl)-8-hydroxychinolin als Farblosen Feststoff. Analog zu Beispiel 2 das Material zu Lithium-4-(2,6-dimethylphenyl)-8-hydroxychinolin umgesetzt und bei 340°C sublimiert.

## Vergleichsbeispiel 1

### Synthese von Lithium 8-hydroxychinolin (V1)

Kommerzielles Lithium 8-hydroxychinolin (Green Fine Chemicals) wird bei 350°C zweimal sublimiert.

## Vergleichsbeispiel 2

### Synthese von Lithium 4-(phenyl)-8-hydroxychinolin (V2)

Analog zu Beispiel 6 wird unter Verwendung von Phenyl-boronsäure Lithium 4-(phenyl)-8-hydroxychinolin hergestellt und bei 380°C sublimiert. Hierbei wird Zersetzung beobachtet.

## Vergleichsbeispiel 3

### Synthese von Lithium 2-(phenyl)-8-hydroxychinolin (V3)

Analog zu Beispiel 3 wird unter Verwendung von Phenyl-boronsäure Lithium 2-(phenyl)-8-hydroxychinolin hergestellt und bei 370°C sublimiert. Hierbei wird Zersetzung beobachtet.

## Beispiel 6

### Mutagenitätsuntersuchungen

Die Verbindungen V1 und E2 werden einem standardisierten AMES Test (Bakterien: Salmonella typhimurium; Stämme TA 98, TA100 und TA 102) unterworfen. Dieser wird mittels DAUN (Daunomycin), $NaN_3$ (Natriumazid), 2-AA (2-Aminoanthracene), B(a)p (Benzo[a]pyren) und CUM (CumolHydroperoxid) referenziert. Die Durchführung des Tests erfolgt durch den Fachmann unter Heranziehung von Standardverfahren, die im Stand der Technik gut bekannt sind. Als Lösungsmittel wird DMSO (Dimethylsulfoxid) verwendet.

Der Test wird sowohl ohne als auch mit metabolischer Aktivierung mit einen S9-Mix durchgeführt.

Hierbei wird für die Verbindung aus Beispiel 2 sowie für weitere erfindungsgemäße Verbindungen keine mutagene Wirkung festgestellt.

Im Gegensatz zu den erfindungsgemäßen Verbindungen zeigt die Referenzverbindung, Liq, eine deutliche Mutagenität in allen drei Bakterienstämmen in Gegenwart der metabolischen Aktivierung.

Die folgende Übersicht fasst die Ergebnisse des Tests für die Vergleichsverbindung, Liq (V1), zusammen.

| Metabol. Akt. | Verbindung | Konz. [μg/plate] | Revertanten/Plate (Mittel±SD) | | |
|---|---|---|---|---|---|
| | | | TA98 | TA100 | TA102 |
| nein | DMSO | | 21±3 | 100±6 | 311±24 |
| nein | Liq | 5 | 23±2 | 96±3 | 344±33 |
| nein | | 15.8 | 20±4 | 99±2 | 270±30 |
| nein | | 50 | 22±1 | 98±4 | 257±59 |
| nein | DAUN | 1 | 175±11 | | |
| nein | $NaN_3$ | 2 | | 1090±35 | |
| nein | CUM | 200 | | | 1481±267 |
| ja | DMSO | | 31±6 | 111±11 | 390±28 |
| ja | Liq | 5 | 31±11 | 146±2 | 559±8 |
| ja | | 15.8 | 40±2 | 261±19 | 697±8 |
| ja | | 50 | 87±1 | 599±115 | 1469±27 |
| ja | | 158 | 100±4 | 788±75 | 911±269 |
| ja | 2-AA | 2 | 201±58 | 384±9 | |
| ja | B(a)p | 10 | | | 2266±18 |
| *Metabol. Akt. - metabolische Aktivierung mit S9-Mix; Konz. - Konzentration; SD - Standardabweichung* | | | | | |

Die folgende Tabelle fasst die Ergebnisse für die erfindungsgemäße Verbindung, E2, zusammen

| Metabol. Akt. | Verbindung | Konz. [μg/plate] | Revertanten/Plate (Mittel ±SD) | | |
|---|---|---|---|---|---|
| | | | TA98 | TA100 | TA102 |
| nein | DMSO | | 18±3 | 128±21 | 216±18 |
| nein | E2 | 5 | 23±11 | 142±25 | 224±6 |
| nein | | 15.8 | 29±3 | 133±8 | 217±41 |
| nein | | 50 | 18±6 | 104±12 | 237±11 |
| nein | | 158 | $20±6^{S}$ | $103±1^{S}$ | $180±11^{S}$ |
| nein | | 500 | $14±8^{SB}$ | $62±17^{SB}$ | $124±1^{SB}$ |
| nein | | 1580 | $10±4^{SB}$ | $41±2^{SBT}$ | $51±18^{SB}$ |
| nein | | 5000 | $17±6^{SE}$ | $7±3^{SET}$ | $25±1^{SE}$ |
| nein | DAUN | 1 | 412±80 | | |
| nein | $NaN_3$ | 2 | | 743±52 | |
| nein | CUM | 200 | | | 912±59 |
| ja | DMSO | | 30±3 | 155±11 | 252±15 |

(fortgesetzt)

| Metabol. Akt. | Verbindung | Konz. [μg/plate] | Revertanten/Plate (Mittel ±SD) | | |
|---|---|---|---|---|---|
| | | | TA98 | TA100 | TA102 |
| ja | E2 | 5 | 24±6 | 199±4 | 302±18 |
| ja | | 15.8 | 30±2 | 132±11 | 255±8 |
| ja | | 50 | 33±4 | 143±8 | 245±22 |
| ja | | 158 | 30±3 S | 147±8 S | 142±22 S |
| ja | | 500 | 25±7 SB | 108±1 SB | 89±11 SB |
| ja | | 1580 | 18±5 SB | 57±16 SBT | 62±3 SB |
| ja | | 5000 | 13±1 SE | 4±3 SET | 37±1 SE |
| ja | 2-AA | 2 | 1376±44 | 1365±18 | |
| ja | B(a)p | 10 | | | 2751±280 |

*S - Plate als Suspension; B - ausgefallen zu Beginn des Experimenfs; E - ausgefallen bis zum Ende des Experiments; T - Toxizität = reduced bacterial background lawn*

**Beispiel 7**

**Charakterisierung des Aufdampfverhaltens**

Das Aufdampfverhalten wird mittels "Effusionsmethode" untersucht. (J-Pestic. Sci. 1982, 13, 161-168). Die Ergebnisse sind in Abbildung 1 zusammengefasst. Die erfindungsgemäßen Verbindungen E2-E5 eisen eine wesentlich bessere Verdampfung auf als die Vergleichsverbindungen V1-V3.

**Beispiel 8**

**Herstellung und Charakterisierung der OLEDs**

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden erfinderischen Beispielen E1 und in den Referenzbeispielen V1 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / p-dotierte Lochtransportschicht A' (HIL1) / Lochtransportschicht A (HTL) / Lochtransportschicht C (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Die zur Herstellung der OLEDs benötigten Materialien sind in Tabelle 1 gezeigt, der Aufbau der verschiedenen hergestellten elektronischen Vorrichtungen in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB(5%) bedeutet hierbei, dass das Material H1 in einem Volumenanteil von 95% und SEB in einem Anteil von 5% in der Schicht vorliegt. Analog kann auch die Elektronentransportschicht oder die Lochinjektionsschichten aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 $cd/m^2$ bestimmt und daraus die CIE 1931 x und y Farbkoordinaten berechnet. Die Angabe EQE @ 10 $mA/cm^2$ bezeichnet die externe Quanteneffizienz bei einer Stromdichte von 10 $mA/cm^2$. LD80 @ 60 $mA/cm^2$ ist die Lebensdauer, bis das OLED bei einem konstanten Strom von 60 $mA/cm^2$ auf 80 % der Anfangsintensität abgefallen ist.

| **Tabelle 1:** Strukturen der verwendeten Materialien | | |
|---|---|---|
| 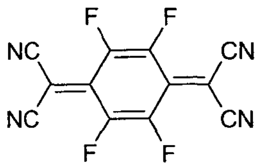 | 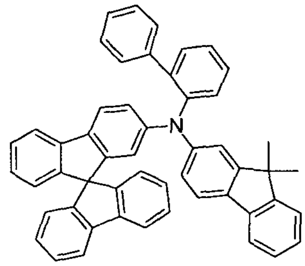 | 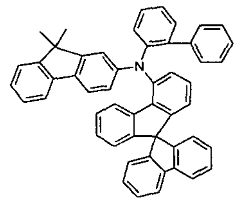 |
| F4TCNQ | HIM | EBM |
| 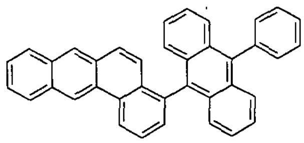 | 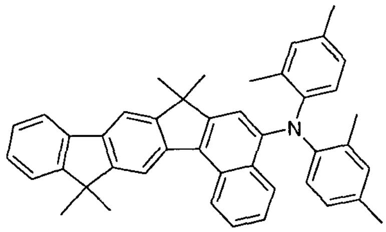 | |
| H1 | SEB | |
| 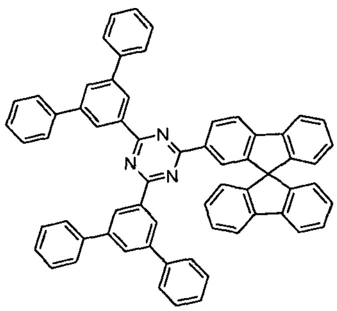 | 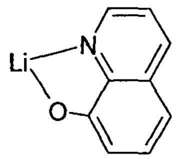 | 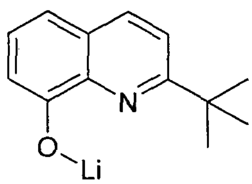 |
| ETM | LiQ | EIM |

**Tabelle 2:**

| *Bsp.* | *HIL1* | *HTL* | *EBL* | *EML* | *ETL* | *EIL* |
|---|---|---|---|---|---|---|
| | *Dicke/nm* | *Dicke/nm* | *Dicke/nm* | *Dicke/nm* | *Dicke/nm* | *Dicke/nm* |
| *V1* | *HIM1:F4TCNQ(5%) 10 nm* | *HIM1 190 nm* | *EBM 10 nm* | *H1:SEB(5%) 20 nm* | *ETM(50%): LiQ(50%) 30 nm* | *LiQ 1 nm* |
| *E1* | *HIM1 :F4TCNQ(5%) 10 nm* | *HIM1 190 nm* | *EBM 10 nm* | *N1: SEB(5%) 20 nm* | *ETM(50%):EIM(50%) 30 nm* | *EIM 2 nm* |
| *E2* | *HIM1:F4TCNQ(5%) 10 nm* | *HIM1 190 nm* | *EBM 10 nm* | *H1:SEB(5%) 20 nm* | *ETM 30 nm* | *EIM 3 nm* |

Die erfindungsgemäße Probe E2 benötigt bei 10 mA/cm$^2$ ungefähr die gleiche Spannung von 3,7 V wie die Referenzprobe V1 mit 3,8 V. Die erfindungsgemäße Probe E1 benötigt eine geringfügig höhere Spannung von 4,3 V als die Referenzprobe. Die Probe E2 hat auch vergleichbare Effizienz von 8.9% EQE bei 10 mA als die Referenzprobe mit 8.9% EQE, während die Probe E1 etwas geringere Effizienz von 8.2 % EQE hat. Probe E1 hat eine etwas bessere Lebensdauer LT80 von 215 h bei 60 mA/cm$^2$ als die Referenzprobe V1. Die Probe E2 hat eine etwas geringere Lebensdauer von 125h. Auch wenn nicht alle Kenndaten zu den LiQ Proben identisch sind, kann gezeigt werden, dass mit dem neuen LiQ-Derivat sehr gute Lebensdauern und hohe Effizienzen erreicht werden können.

Dabei ist festzustellen, dass die hier verwendeten OLEDs keine optimierten Vorrichtungen darstellen und dem

Fachmann ist es ohne erfinderisches Zutun möglich, die Effizienz der OLEDs durch geeignete, ihm geläufige Maßnahmen zu steigern. Eine solche Steigerung der Effizienz kann, beispielsweise, dann beobachtet werden, wenn erfindungsgemäße Zusammensetzungen anstelle der Einzelverbindungen in den entsprechenden Schichten eingesetzt werden.

**Patentansprüche**

1. Verbindung gemäß Formel (1)

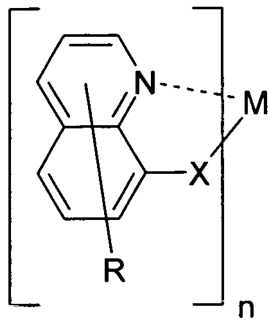

Formel (1)

wobei

M ist Li;
X ist S oder O, bevorzugt O;
R eine verzweigte oder cyclische Alkyl- oder Alkoxygruppe mit 3 bis 40 C-Atomen, die jeweils mit einem oder mehreren Resten $R^a$ substituiert sein kann, wobei eine oder mehrere nicht benachbarte $CH_2$-Gruppen durch $R^aC=CRa$, C≡C, $Si(R^a)_2$, $Ge(R^a)_2$, $Sn(R^a)_2$, C=O, C=S, C=Se, $C=NR^a$, $P(=O)(R^a)$, SO, $SO_2$, $NR^a$, O, S oder $CONR^a$ ersetzt sein können und wobei ein oder mehrere H-Atome durch D, F, Cl, Br, I, CN oder $NO_2$ ersetzt sein können, oder ein aromatisches oder heteroaromatisches Ringsystem, bevorzugt ein aromatisches Ringsystem, mit 5 bis 60 aromatischen Ringatomen, das zumindest in einer ortho-Position in Bezug auf die Bindungsstelle an den Chinolinring einen Rest $R^b$ aufweist und gegebenenfalls durch einen oder mehrere Reste $R^a$ substituiert sein kann, wobei der Rest R bevorzugt kein Ringsystem zusammen mit dem Chinolinring bildet;
$R^a$ bei jedem Auftreten gleich oder verschieden H, D oder eine Alkylgruppe mit 1 bis 20 C-Atomen, ein aromatisches Ringsystem mit 6 bis 60 C-Ring-Atomen oder ein heteroaromatisches Ringsystem mit 1 bis 60 C-Ring-Atomen ist, in dem auch H-Atome durch D oder F ersetzt sein können; dabei können zwei oder mehrere benachbarte Substituenten $R^a$ auch miteinander ein mono- oder polycyclisches, aliphatisches, aromatisches oder heteroaromatisches Ringsystem bilden wobei bevorzugt ist, wenn zwei oder mehrere benachbarte Reste $R^a$ keinen Ringschluss bilden;
$R^b$ bei jedem Auftreten gleich oder verschieden eine Alkylgruppe mit 1 bis 20 C-Atomen, ein aromatisches Ringsystem mit 6 bis 60 C-Ring-Atomen oder heteroaromatisches Ringsystem mit 1 bis 60 C-Ring-Atomen, wobei H-Atome durch D oder F ersetzt sein können; dabei können zwei oder mehrere benachbarte Substituenten $R^b$ auch miteinander ein mono- oder polycyclisches, aliphatisches oder aromatisches Ringsystem bilden, wobei bevorzugt ist, wenn zwei oder mehrere benachbarte Reste $R^b$ keinen Ringschluss bilden
n ist 1 ;

mit der Maßgabe, dass wenn R ein aromatisches Ringsystem ist, das in nur einer ortho-Position zum Chinolinring einen Rest $R^b$ aufweist, der Rest $R^b$ mindestens 2 C-Atome, bevorzugt mindestens 3 C-Atome und ganz bevorzugt mindestens 4 C-Atome aufweist
und wobei R an Position 2 an den Chinolinring gebunden ist.

2. Verbindung gemäß Anspruch 1, dadurch charakterisiert, dass R eine verzweigte Alkylgruppe mit 4 bis 40 C-Atomen, bevorzugt mit 4 bis 30 C-Atomen, ganz bevorzugt mit 4 bis 20 C-Atomen, ganz besonders bevorzugt mit 4 bis 10 C-Atomen und insbesondere bevorzugt mit 4 bis 6 C-Atomen ist, eine cyclische Alkylgruppe mit 3 bis 40 C-Atomen, bevorzugt mit 3 bis 30 C-Atomen, ganz bevorzugt mit 3 bis 20 C-Atomen, ganz besonders bevorzugt mit 3 bis 15 C-Atomen und insbesondere bevorzugt mit 6 bis 12 C-Atomen ist, wobei die Gruppen jeweils mit einem oder

mehreren Resten $R^a$ substituiert sein können und wobei ein oder mehrere H-Atome durch D, F, Cl, Br, I, CN oder $NO_2$ ersetzt sein können, oder ein aromatisches Ringsystem mit 5 bis 60 aromatischen Ringatomen, das zumindest in einer ortho-Position in Bezug auf die Bindungsstelle an den Chinolinring einen Rest $R^b$ aufweist und gegebenenfalls durch einen oder mehrere Reste $R^a$ substituiert sein kann, wobei bevorzugt keiner der Reste R, $R^a$ oder $R^b$ mit dem Chinolinring ein Ringsystem bildet.

3. Verbindung gemäß Anspruch 1 oder 2, **dadurch gekennzeichnet, dass** $R^a$ bei jedem Auftreten gleich oder verschieden H, D oder eine Alkylgruppe mit 1 bis 20 C-Atomen ist, wobei zwei oder mehrere benachbarte Reste $R^a$ keinen Ringschluss bilden.

4. Verbindung gemäß einem oder mehreren der Ansprüche 1 bis 3, **dadurch gekennzeichnet, dass** der Rest R eine der folgenden Formeln aufweist, wobei die gestrichelten Linien die Bindung zu dem Chinolin der Formel (1) kennzeichnen

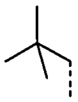

(R-1)

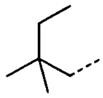

(R-2)

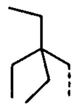

(R-3)

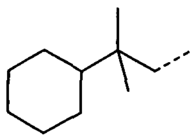

(R-4)

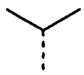

(R-5)

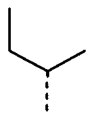

(R-6)

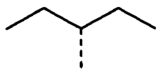

(R-7)

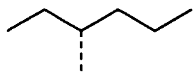

(R-8)

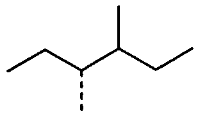

(R-9)

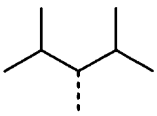

(R-10)

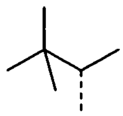

(R-11)

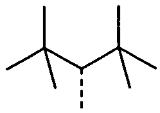

(R-12)

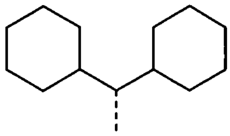

(R-13)

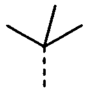

(R-14)

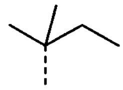

(R-15)

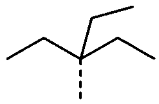

(R-16)

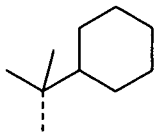

(R-17)

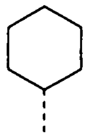

(R-18)

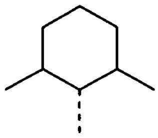

(R-19)

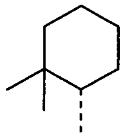

(R-20)

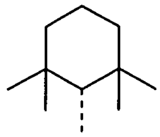

(R-21)

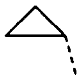

(R-22)

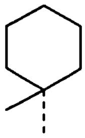

(R-23)

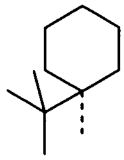

(R-24)

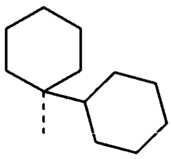

(R-25)

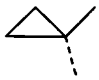

(R-26)

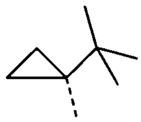

(R-27)

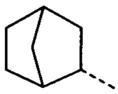

(R-28)

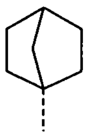

(R-29)

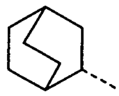

(R-30)

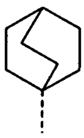

(R-31)

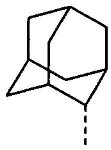

(R-32)

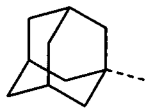

(R-33)

**5.** Verbindung gemäß einem oder mehreren der Ansprüche 1 bis 4, dadurch charakterisiert, dass R über ein quartäres Kohlenstoffatom an den Chinolinring gebunden ist.

6. Verbindung gemäß einem oder mehreren der Ansprüche 1 bis 5, **dadurch gekennzeichnet, dass** der Rest $R^a$ gleich H ist.

7. Verbindung gemäß einem oder mehreren der Ansprüche 1 bis 6, **dadurch gekennzeichnet, dass** es sich um eine Verbindung mit den folgenden Formeln handelt

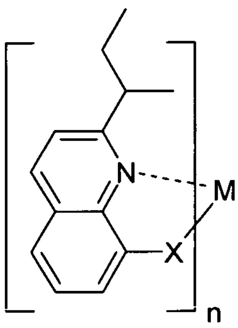

Formel (A-2)

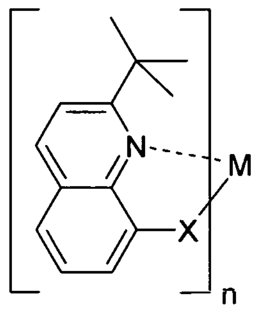

Formel (A-3)

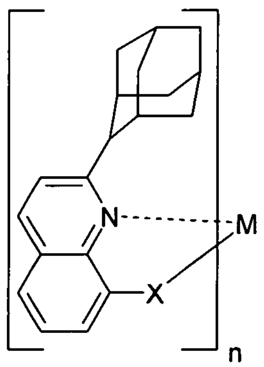

Formel (A-4)

8. Verbindung gemäß einem oder mehreren der Ansprüche 1 bis 3, **dadurch gekennzeichnet, dass** $R^b$ bei jedem Auftreten gleich oder verschieden eine Alkylgruppe mit 1 bis 20 C-Atomen ist, wobei bevorzugt ist wenn zwei oder mehrere benachbarte Reste $R^b$ keinen Ringschluss bilden.

9. Verbindung gemäß einem oder mehreren der Ansprüche 1 bis 3 oder 9, **dadurch gekennzeichnet, dass** es sich um eine Verbindung der folgenden Formeln handelt

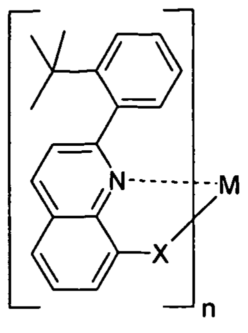

Formel (B-1)

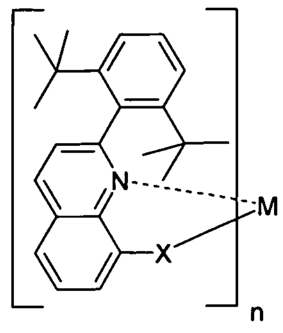

Formel (B-2)

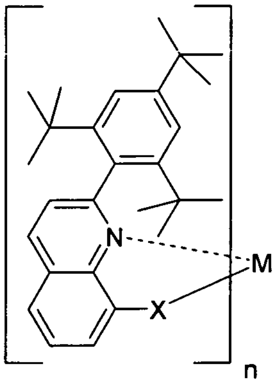

Formel (B-2)

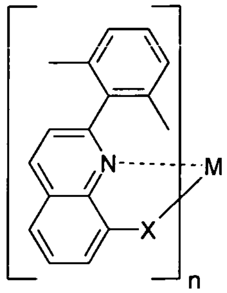

Formel (B-3)

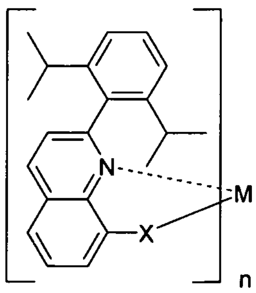

Formel (B-4)

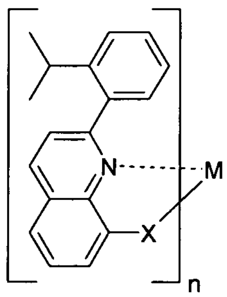

Formel (B-5)

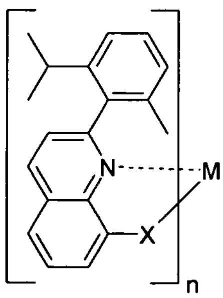

Formel (B-6)

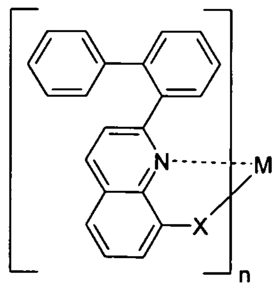

Formel (B-7)

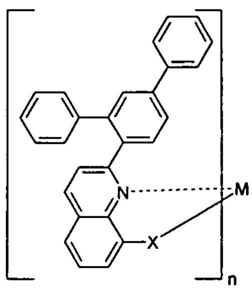

Formel (B-8)

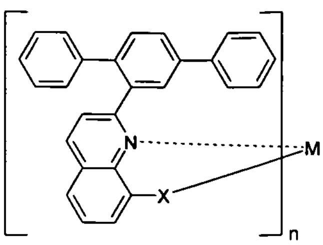

Formel (B-9)

**10.** Zusammensetzung enthaltend eine oder mehrere Verbindungen gemäß einem oder mehreren der Ansprüche 1 bis 9 sowie mindestens ein zusätzliches funktionelles Molekül ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien, Lochblockiermaterialien und n-Dotanden.

**11.** Zusammensetzung gemäß Anspruch 10, dadurch charakterisiert, dass es sich bei dem zusätzlichen funktionellen Material um ein Elektronentransportmaterial handelt, das bevorzugt ausgewählt ist aus Pyridinen, Pyrimidinen, Pyridazinen, Pyrazinen, Oxadiazolen, Oxazolen, Lactamen, Chinolinen, Chinoxalinen, Anthracenen, Benzanthracenen, Pyrenen, Perylenen, Benzimidazolen, Triazinen, Ketonen, Phosphinoxiden und Phenazinen, ganz bevorzugt aus Triazinen.

**12.** Zusammensetzung gemäß Anspruch 10 oder 11, **dadurch gekennzeichnet, dass** es sich bei dem zusätzlichen funktionellen Material um ein Elektronentransportmaterial handelt, das eine Verbindung der folgenden allgemeinen Formel (2) aufweist

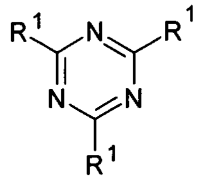

Formel 2

wobei für die verwendeten Symbole und Indizes gilt:

$R^1$ ist bei jedem Auftreten gleich oder verschieden H, D, F, Cl, Br, I, CHO, $N(R^2)_2$, $N(Ar^1)_2$, $B(Ar^1)_2$, $C(=O)Ar^1$, $P(=O)(Ar^1)_2$, $S(=O)Ar^1$, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, eine geradkettige Alkyl-, Alkenyl-, Alkinyl-, Alkoxy- oder Thioalkoxygruppe mit 1 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren Resten $R^2$ substituiert sein kann; dabei können auch zwei Reste $Ar^1$, welche an dasselbe Stickstoff-, Phosphor- oder Boratom binden, durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verknüpft sein;

$R^2$ ist bei jedem Auftreten gleich oder verschieden H, D oder ein aliphatischer, aromatischer und/oder heteroaromatischer Kohlenwasserstoffrest mit 1 bis 20 C-Atomen, in dem auch H-Atome durch D oder F ersetzt sein können; dabei können zwei oder mehrere benachbarte Substituenten $R^2$ auch miteinander ein mono- oder polycyclisches, aliphatisches oder aromatisches Ringsystem bilden.

**13.** Zusammensetzung gemäß Anspruch 10, dadurch charakterisiert, dass es sich bei dem zusätzlichen funktionellen Material um einen n-Dotanden handelt.

**14.** Formulierung enthaltend mindestens eine Verbindung gemäß einem oder mehreren der Ansprüche 1 bis 9 oder mindestens eine Zusammensetzung gemäß einem oder mehreren der Ansprüche 10 bis 13 sowie mindestens ein Lösungsmittel.

**15.** Verwendung mindestens einer Verbindung gemäß einem oder mehreren der Ansprüche 1 bis 19 oder mindestens einer Zusammensetzung gemäß einem oder mehreren der Ansprüche 10 bis 13 in einer elektronischen Vorrichtung, bevorzugt in einer organischen Elektromunieszenzvorrichtung, ganz bevorzugt in einer organischen lichtemittierenden Diode (OLED) oder in einer organischen lichtemittierenden elektrochemischen Zelle (OLEC, LEC oder auch LEEC), ganz besonders bevorzugt in einer OLED.

**16.** Vorrichtung enthaltend mindestens eine Verbindung gemäß einem oder mehreren der Ansprüche 1 bis 9 oder

mindestens einer Zusammensetzung gemäß einem oder mehreren der Ansprüche 10 bis 13.

**17.** Vorrichtung gemäß Anspruch 16, dadurch charakterisiert, dass es sich bei der Vorrichtung um eine elektronische Vorrichtung handelt.

**18.** Vorrichtung gemäß Anspruch 16 oder 17, dadurch charakterisiert, dass es sich bei der Vorrichtung um eine elektronische Vorrichtung handelt, die ausgewählt ist aus der Gruppe bestehend aus den organischen Elektrolumineszenzvorrichtungen, den organischen integrierten Schaltungen; organischen Feld-Effekt-Transistoren, organischen Dünnfilmtransistoren; organischen Solarzellen; organischen optischen Detektoren; organischen Photorezeptoren oder organischen Feld-Quench-Devices, wobei die organischen Elektrolumineszenzvorrichtungen bevorzugt sind.

**19.** Vorrichtung gemäß einem oder mehreren der Ansprüche 16 bis 18, **dadurch gekennzeichnet, dass** es sich um eine organische Elektrolumineszenzvorrichtung handelt, die ausgewählt ist aus der Gruppe bestehend aus den organischen lichtemittierenden Dioden (OLED), den organischen lichtemittierenden Transistoren, den organischen lichtemittierenden elektrochemischen Zellen (OLEC, LEC oder auch LEEC) und den organischen Laserdioden, bevorzugt aus den OLEDs und OLECs und ganz bevorzugt aus den OLEDs.

**20.** Vorrichtung gemäß einem oder mehreren der Ansprüche 16 bis 19, **dadurch gekennzeichnet, dass** sie die mindestens eine Verbindung gemäß einem oder mehreren der Ansprüche 1 bis 9 oder die mindestens eine Zusammensetzung gemäß einem oder mehreren der Ansprüche 10 bis 13 in einer elektronenleitenden Schicht enthält.

**21.** Vorrichtung gemäß einem oder mehreren der Ansprüche 16 bis 20, **dadurch gekennzeichnet, dass** sie die mindestens eine Verbindung gemäß einem oder mehreren der Ansprüche 1 bis 9 oder die mindestens eine Zusammensetzung gemäß einem oder mehreren der Ansprüche 10 bis 13 in einer Elektroneninjektionsschicht oder in einer Elektronentransportschicht enthält.

**22.** Verfahren zur Herstellung der Verbindung gemäß einem oder mehreren der Ansprüche 1 bis 9 durch Herstellung des Liganden ohne Metall und anschließender Umsetzung des Liganden mit einem Metallsalz, um den Metallkomplex gemäß einem oder mehreren der Ansprüche 1 bis 9 zu erhalten.

**23.** Elektrolumineszierende Vorrichtung, bevorzugt eine OLED oder OLEC, enthaltend mindestens eine Verbindung gemäß einem oder mehreren der Ansprüche 1 bis 9 oder mindestens eine Zusammensetzung gemäß einem oder mehreren der Ansprüche 10 bis 13 zur Verwendung in der Medizin.

**24.** Kosmetische Verwendung einer elektrolumineszierenden Vorrichtung, bevorzugt einer OLED oder OLEC, enthaltend mindestens eine Verbindung gemäß einem oder mehreren der Ansprüche 1 bis 9 oder mindestens eine Zusammensetzung gemäß einem oder mehreren der Ansprüche 10 bis 13.

## Claims

**1.** Compound of the formula (1)

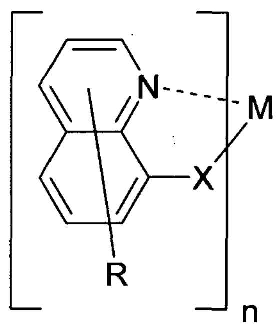

formula (1)

where

M is Li;

X is S or O, preferably O;

R is a branched or cyclic alkyl or alkoxy group having 3 to 40 C atoms, which may in each case be substituted by one or more radicals $R^a$, where one or more non-adjacent $CH_2$ groups may be replaced by $R^aC=CR^a$, C≡C, $Si(R^a)_2$, $Ge(R^a)_2$, $Sn(R^a)_2$, C=O, C=S, C=Se, $C=NR^a$, $P(=O)(R^a)$, SO, $SO_2$, $NR^a$, O, S or $CONR^a$ and where one or more H atoms may be replaced by D, F, Cl, Br, I, CN or $NO_2$, or an aromatic or heteroaromatic ring system, preferably an aromatic ring system, having 5 to 60 aromatic ring atoms which contains a radical $R^b$ at least in one ortho position relative to the bonding site on the quinoline ring and may optionaally be substituted by one or more radicals $R^a$, where the radical R preferably does not form a ring system together with the quinoline ring;

$R^a$ is on each occurrence, identically or differently, H, D or an alkyl group having 1 to 20 C atoms, an aromatic ring system having 6 to 60 C ring atoms or a heteroaromatic ring system having 1 to

$R^b$ 60 C ring atoms, in which, in addition, H atoms may be replaced by D or F; two or more adjacent substituents $R^a$ here may also form a mono- or polycyclic, aliphatic, aromatic or heteroaromatic ring system with one another, where it is preferred for two or more adjacent radicals $R^a$ not to form a ring closure; is on each occurrence, identically or differently, an alkyl group having 1 to 20 C atoms, an aromatic ring system having 6 to 60 C ring atoms or a heteroaromatic ring system having 1 to 60 C ring atoms, where H atoms may be replaced by D or F; two or more adjacent substituents $R^b$ here may also form a mono- or polycyclic, aliphatic or aromatic ring system with one another, where it is preferred for two or more adjacent radicals $R^b$ not to form a ring closure;

n is 1;

with the proviso that, if R is an aromatic ring system which contains a radical $R^b$ in only one ortho position to the quinoline ring, the radical $R^b$ has at least 2 C atoms, preferably at least 3 C atoms and very preferably at least 4 C atoms,

and where R is bonded to the quinoline ring at position 2.

2. Compound according to Claim 1, **characterised in that** R is a branched alkyl group having 4 to 40 C atoms, preferably having 4 to 30 C atoms, very preferably having 4 to 20 C atoms, very particularly preferably having 4 to 10 C atoms and especially preferably having 4 to 6 C atoms, a cyclic alkyl group having 3 to 40 C atoms, preferably having 3 to 30 C atoms, very preferably having 3 to 20 C atoms, very particularly preferably having 3 to 15 C atoms and especially preferably having 6 to 12 C atoms, where the groups may each be substituted by one or more radicals $R^a$ and where one or more H atoms may be replaced by D, F, Cl, Br, I, CN or $NO_2$, or an aromatic ring system having 5 to 60 aromatic ring atoms which contains a radical $R^b$ at least in one ortho position relative to the bonding site on the quinoline ring and may optionally be substituted by one or more radicals $R^a$, where preferably none of the radicals R, $R^a$ or $R^b$ forms a ring system with the quinoline ring.

3. Compound according to Claim 1 or 2, **characterised in that** $R^a$ is on each occurrence, identically or differently, H, D or an alkyl group having 1 to 20 C atoms, where two or more adjacent radicals $R^a$ do not form a ring closure.

4. Compound according to one or more of Claims 1 to 3, **characterised in that** the radical R has one of the following formulae, where the dashed lines denote the bond to the quinoline of the formula (1):

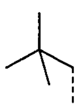

(R-1)

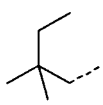

(R-2)

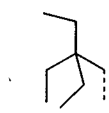

(R-3)

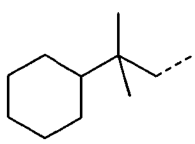

(R-4)

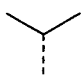

(R-5)

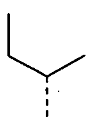

(R-6)

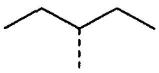
(R-7)

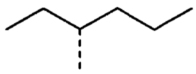
(R-8)

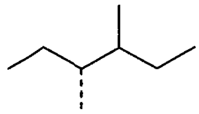
(R-9)

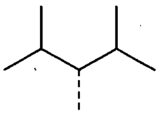
(R-10)

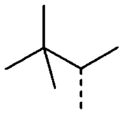
(R-11)

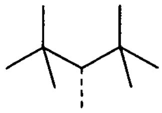
(R-12)

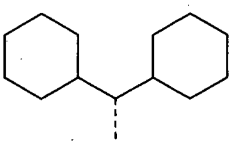
(R-13)

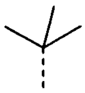
(R-14)

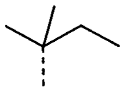
(R-15)

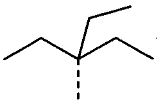
(R-16)

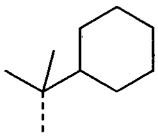
(R-17)

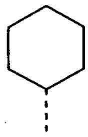
(R-18)

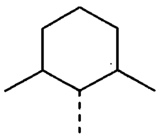
(R-19)

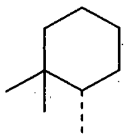
(R-20)

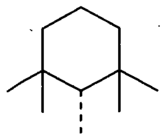
(R-21)

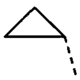
(R-22)

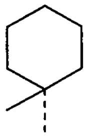
(R-23)

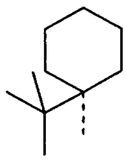
(R-24)

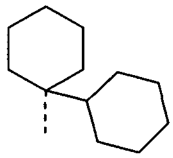
(R-25)

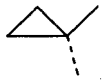
(R-26)

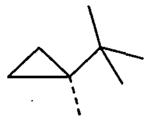
(R-27)

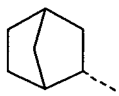
(R-28)

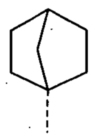
(R-29)

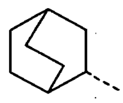
(R-30)

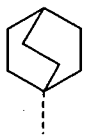
(R-31)

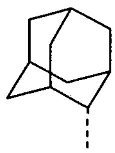
(R-32)

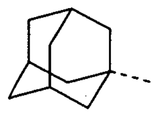
(R-33)

5. Compound according to one or more of Claims 1 to 4, **characterised in that** R is bonded to the quinoline ring via a quaternary carbon atom.

6. Compound according to one or more of Claims 1 to 5, **characterised in that** the radical $R^a$ is equal to H.

7. Compound according to one or more of Claims 1 to 6, **characterised in that** it is a compound having the following formulae:

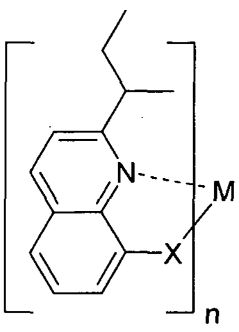
formula (A-2)

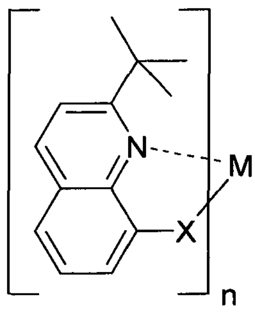
formula (A-3)

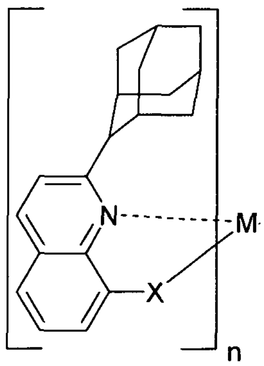

formula (A-4)

8. Compound according to one or more of Claims 1 to 3, **characterised in that** $R^b$ is on each occurrence, identically or differently, an alkyl group having 1 to 20 C atoms, where it is preferred for two or more adjacent radicals $R^b$ not to form a ring closure.

9. Compound according to one or more of Claims 1 to 3 or 8, **characterised in that** it is a compound of the following formulae:

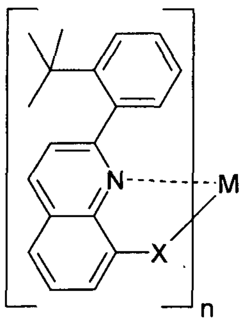

formula (B-1)

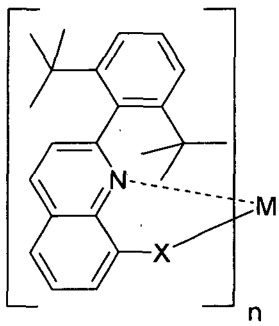

formula (B-2)

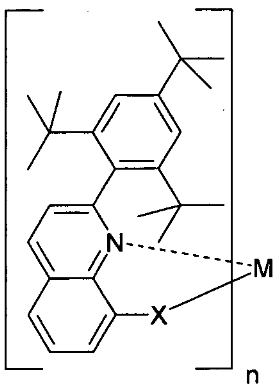

formula (B-2)

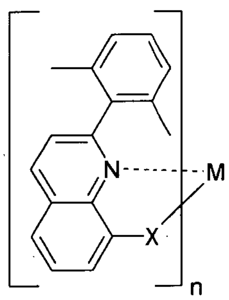

formula (B-3)

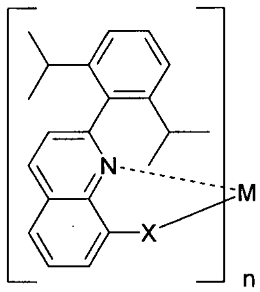

formula (B-4)

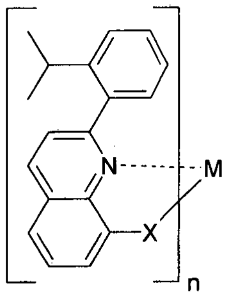

formula (B-5)

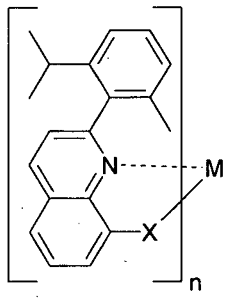

formula (B-6)

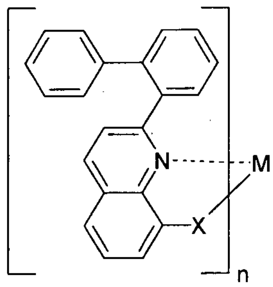

formula (B-7)

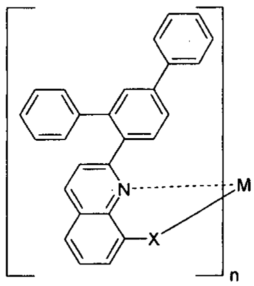

formula (B-8)

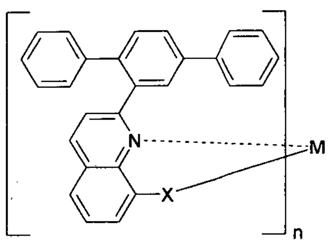

formula (B-9)

**10.** Composition comprising one or more compounds according to one or more of Claims 1 to 9 and at least one additional functional molecule selected from the group consisting of fluorescent emitters, phosphorescent emitters, host materials, matrix materials, electron-transport materials, electron-injection materials, hole-conductor materials, hole-injection materials, electron-blocking materials, hole-blocking materials and n-dopants.

**11.** Composition according to Claim 10, **characterised in that** the additional functional material is an electron-transport material, which is preferably selected from pyridines, pyrimidines, pyridazines, pyrazines, oxadiazoles, oxazoles, lactams, quinolines, quinoxalines, anthracenes, benzanthracenes, pyrenes, perylenes, benzimidazoles, triazines, ketones, phosphine oxides and phenazines, very preferably from triazines.

**12.** Composition according to Claim 10 or 11, **characterised in that** the additional functional material is an electron-transport material which comprises a compound of the following general formula (2):

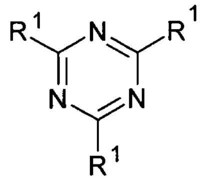

formula (2)

where the following applies to the symbols and indices used:

$R^1$ is on each occurrence, identically or differently, H, D, F, Cl, Br, I, CHO, $N(R^2)_2$, $N(Ar^1)_2$, $B(Ar^1)_2$, $C(=O)Ar^1$, $P(=O)(Ar^1)_2$, $S(=O)Ar^1$, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, a straight-chain alkyl, alkenyl, alkynyl, alkoxy or thioalkoxy group having 1 to 40 C atoms or a branched or cyclic alkyl, alkenyl, alkynyl, alkoxy or thioalkoxy group having 3 to 40 C atoms, which may in each case be substituted by one or more radicals $R^2$, where one or more non-adjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy or heteroaryloxy group having 5 to 60 aromatic ring atoms, which may be substituted by one or more radicals $R^2$, or a combination of these systems; two or more adjacent substituents $R^2$ here may also form a mono- or polycyclic, aliphatic or aromatic ring system with one another;

$Ar^1$ is on each occurrence, identically or differently, an aromatic or heteroaromatic ring system having 5 to 30 aromatic ring atoms, which may be substituted by one or more radicals $R^2$; two radicals $Ar^1$ which are bonded to the same nitrogen, phosphorus or boron atom may also be linked to one another here by a single bond or a bridge selected from $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ and $P(=O)R^2$;

$R^2$ is on each occurrence, identically or differently, H, D or an aliphatic, aromatic and/or heteroaromatic hydrocarbon radical having 1 to 20 C atoms, in which, in addition, H atoms may be replaced by D or F; two or more adjacent substituents $R^2$ here may also form a mono- or polycyclic, aliphatic or aromatic ring system with one another.

**13.** Composition according to Claim 10, **characterised in that** the additional functional material is an n-dopant.

**14.** Formulation comprising at least one compound according to one or more of Claims 1 to 9 or at least one composition according to one or more of Claims 10 to 13 and at least one solvent.

**15.** Use of at least one compound according to one or more of Claims 1 to 9 or at least one composition according to one or more of Claims 10 to 13 in an electronic device, preferably in an organic electroluminescent device, very preferably in an organic light-emitting diode (OLED) or in an organic light-emitting electrochemical cell (OLEC, LEC or also LEEC), very particularly preferably in an OLED.

**16.** Device containing at least one compound according to one or more of Claims 1 to 9 or at least one composition according to one or more of Claims 10 to 13.

**17.** Device according to Claim 16, **characterised in that** the device is an electronic device.

**18.** Device according to Claim 16 or 17, **characterised in that** the device is an electronic device which is selected from the group consisting of organic electroluminescent devices, organic integrated circuits, organic field-effect transistors, organic thin-film transistors, organic solar cells, organic optical detectors, organic photoreceptors and organic field-quench devices, where organic electroluminescent devices are preferred.

**19.** Device according to one or more of Claims 16 to 18, **characterised in that** it is an organic electroluminescent device which is selected from the group consisting of organic light-emitting diodes (OLEDs), organic light-emitting transistors, organic light-emitting electrochemical cells (OLECs, LECs or also LEECs) and organic laser diodes, preferably from OLEDs and OLECs and very preferably from OLEDs.

**20.** Device according to one or more of Claims 16 to 19, **characterised in that** it contains the at least one compound according to one or more of Claims 1 to 9 or the at least one composition according to one or more of Claims 10 to

13 in an electron-conducting layer.

**21.** Device according to one or more of Claims 16 to 20, **characterised in that** it contains the at least one compound according to one or more of Claims 1 to 9 or the at least one composition according to one or more of Claims 10 to 13 in an electron-injection layer or in an electron-transport layer.

**22.** Process for the preparation of the compound according to one or more of Claims 1 to 9 by preparation of the ligand without metal and subsequent reaction of the ligand with a metal salt in order to obtain the metal complex according to one or more of Claims 1 to 9.

**23.** Electroluminescent device, preferably an OLED or OLEC, containing at least one compound according to one or more of Claims 1 to 9 or at least one composition according to one or more of Claims 10 to 13 for use in medicine.

**24.** Cosmetic use of an electroluminescent device, preferably an OLED or OLEC, containing at least one compound according to one or more of Claims 1 to 9 or at least one composition according to one or more of Claims 10 to 13.

**Revendications**

**1.** Composé de la formule (1) :

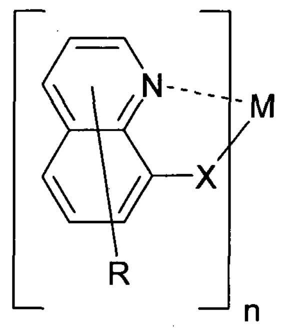

formule (1)

dans laquelle :

M est Li ;
X est S ou O, de façon préférable O ;
R est un groupe alkyle ou alcoxy ramifié ou cyclique qui comporte 3 à 40 atomes de C, lequel peut, dans chaque cas, être substitué par un radical ou plusieurs radicaux $R^a$, où un ou plusieurs groupe(s) $CH_2$ non adjacents peut/peuvent être remplacé(s) par $R^aC=CR^a$, C≡C, $Si(R^a)_2$, $Ge(R^a)_2$, $Sn(R^a)_2$, C=O, C=S, C=Se, $C=NR^a$, $P(=O)(R^a)$, SO, $SO_2$, $NR^a$, O, S ou $CONR^a$ et où un ou plusieurs atome(s) de H peut/peuvent être remplacé(s) par D, F, Cl, Br, I, CN ou $NO_2$, ou un système de cycle aromatique ou hétéroaromatique, de façon préférable un système de cycle aromatique, qui comporte 5 à 60 atomes de cycle aromatique, lequel contient un radical $R^b$ au moins au niveau d'une position ortho par rapport au site de liaison sur le cycle quinoline et peut en option être substitué par un radical ou plusieurs radicaux $R^a$, où le radical R, de façon préférable, ne forme pas un système de cycle en association avec le cycle quinoline ;
$R^a$ est, pour chaque occurrence, de manière identique ou différente, H, D ou un groupe alkyle qui comporte 1 à 20 atome(s) de C, un système de cycle aromatique qui comporte 6 à 60 C atomes de cycle ou un système de cycle qui comporte 1 à 60 C atome(s) de cycle, où, en outre, des atomes de H peuvent être remplacés par D ou F ; deux substituants adjacents ou plus $R^a$ ici peuvent également former un système de cycle monocyclique ou polycyclique, aliphatique, aromatique ou hétéroaromatique l'un avec l'autre ou les uns avec les autres, où il est préférable que deux radicaux $R^a$ adjacents ou plus ne forment pas une fermeture de cycle ;
$R^b$ est, pour chaque occurrence, de manière identique ou différente, un groupe alkyle qui comporte 1 à 20 atome(s) de C, un système de cycle aromatique qui comporte 6 à 60 atomes de cycle C ou un système de cycle hétéroaromatique qui comporte 1 à 60 atome(s) de cycle C, où des atomes de H peuvent être remplacés par D ou F ; deux substituants $R^b$ adjacents ou plus ici peuvent également former un système de cycle monocyclique ou polycyclique, aliphatique ou aromatique l'un avec l'autre ou les uns avec les autres, où il est préférable que deux radicaux $R^b$ adjacents ou plus ne forment pas une fermeture de cycle ;
n est 1 ;

étant entendu que si R est un système de cycle aromatique qui contient un radical $R^b$ seulement au niveau de la position ortho sur le cycle quinoline, le radical $R^b$ comporte au moins 2 atomes de C, de façon préférable au moins 3 atomes de C et de façon très préférable, au moins 4 atomes de C,
et où R est lié sur le cycle quinoline au niveau de la position 2.

**2.** Composé selon la revendication 1, **caractérisé en ce que** R est un groupe alkyle ramifié qui comporte 4 à 40 atomes de C, de façon préférable, qui comporte 4 à 30 atomes de C, de façon très préférable, qui comporte 4 à 20 atomes de C, de façon très particulièrement préférable, qui comporte 4 à 10 atomes de C et de façon tout particulièrement préférable, qui comporte 4 à 6 atomes de C, un groupe alkyle cyclique qui comporte 3 à 40 atomes de C, de façon préférable, qui comporte 3 à 30 atomes de C, de façon très préférable, qui comporte 3 à 20 atomes de C, de façon très particulièrement préférable, qui comporte 3 à 15 atomes de C et de façon tout particulièrement préférable, qui comporte 6 à 12 atomes de C, où les groupes peuvent chacun être substitués par un radical ou plusieurs radicaux $R^a$ et où un ou plusieurs atome(s) de H peut/peuvent être remplacé(s) par D, F, Cl, Br, I, CN ou $NO_2$, ou un système de cycle aromatique qui comporte 5 à 60 atomes de cycle aromatique, lequel contient un radical $R^b$ au moins au niveau d'une position ortho par rapport au site de liaison sur le cycle quinoline et peut en option être substitué par un radical ou plusieurs radicaux $R^a$, où de façon préférable, aucun des radicaux R, $R^a$ ou $R^b$ ne forme un système de cycle avec le cycle quinoline.

**3.** Composé selon la revendication 1 ou 2, **caractérisé en ce que** $R^a$ est pour chaque occurrence, de manière identique ou différente, H, D ou un groupe alkyle qui comporte 1 à 20 atome(s) de C, où deux radicaux $R^a$ adjacents ou plus ne forment pas une fermeture de cycle.

**4.** Composé selon une ou plusieurs des revendications 1 à 3, **caractérisé en ce que** le radical R présente l'une des formules qui suivent, dans lesquelles les lignes en pointillés représentent la liaison sur la quinoline de la formule (1) :

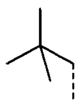
(R-1)

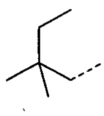
(R-2)

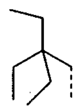
(R-3)

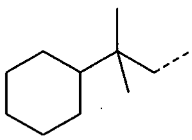
(R-4)

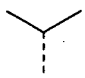
(R-5)

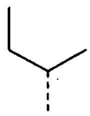
(R-6)

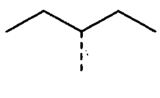
(R-7)

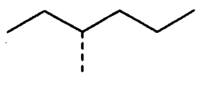
(R-8)

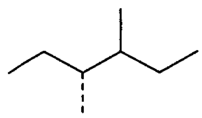
(R-9)

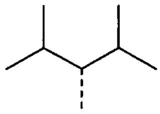

(R-10)

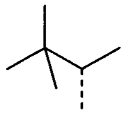

(R-11)

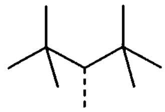

(R-12)

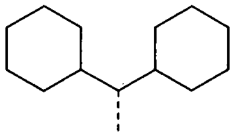

(R-13)

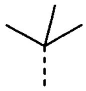

(R-14)

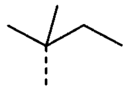

(R-15)

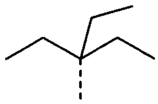

(R-16)

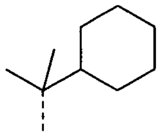

(R-17)

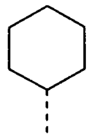

(R-18)

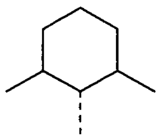

(R-19)

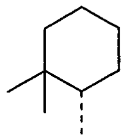

(R-20)

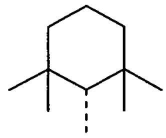

(R-21)

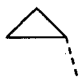

(R-22)

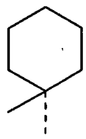

(R-23)

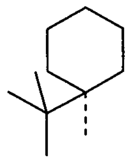

(R-24)

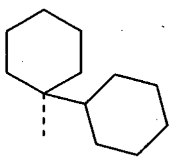

(R-25)

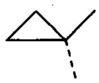

(R-26)

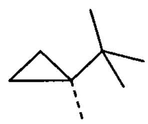

(R-27)

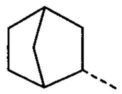

(R-28)

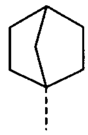

(R-29)

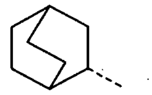

(R-30)

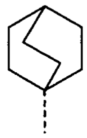

(R-31)

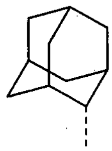

(R-32)

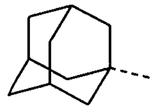

(R-33)

5. Composé selon une ou plusieurs des revendications 1 à 4, **caractérisé en ce que** R est lié sur le cycle quinoline via un atome de carbone quaternaire.

6. Composé selon une ou plusieurs des revendications 1 à 5, **caractérisé en ce que** le radical $R^a$ est égal à H.

7. Composé selon une ou plusieurs des revendications 1 à 6, **caractérisé en ce qu'**il s'agit d'un composé qui présente les formules qui suivent :

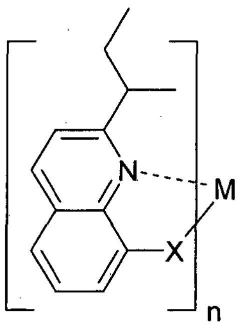

formule (A-2)

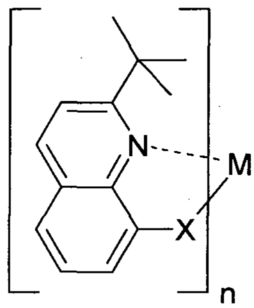

formule (A-3)

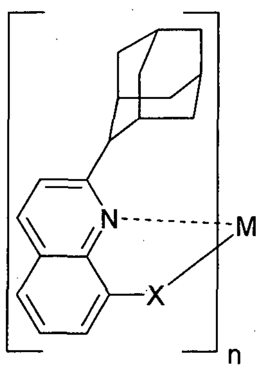

formule (A-4)

8. Composé selon une ou plusieurs des revendications 1 à 3, **caractérisé en ce que** $R^b$ est pour chaque occurrence, de manière identique ou différente, un groupe alkyle qui comporte 1 à 20 atome(s) de C, où il est préférable que deux radicaux $R^b$ adjacents ou plus ne forment pas une fermeture de cycle.

**9.** Composé selon une ou plusieurs des revendications 1 à 3 ou 8, **caractérisé en ce qu'**il s'agit d'un composé des formules qui suivent :

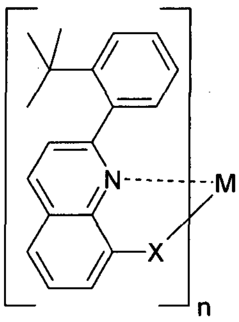

formule (B-1)

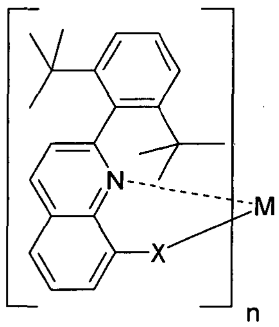

formule (B-2)

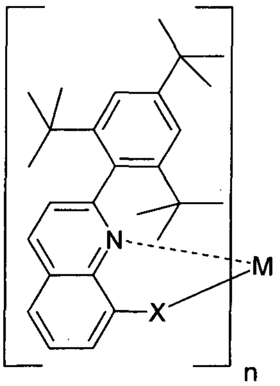

formule (B-2)

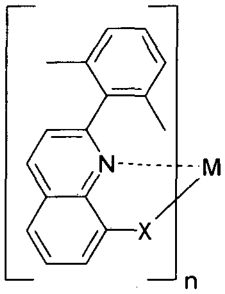

formule (B-3)

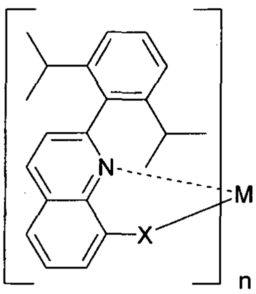

formule (B-4)

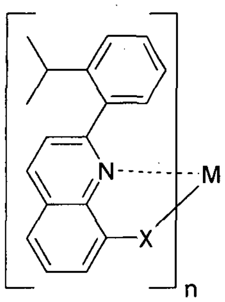

formule (B-5)

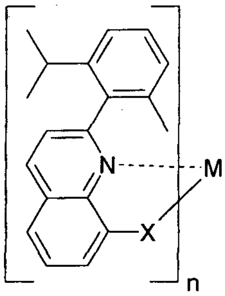

formule (B-6)

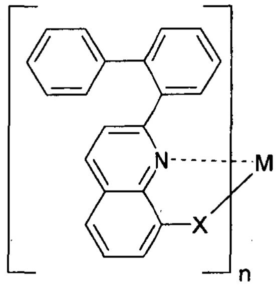

formule (B-7)

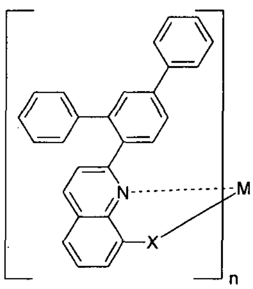

formule (B-8)

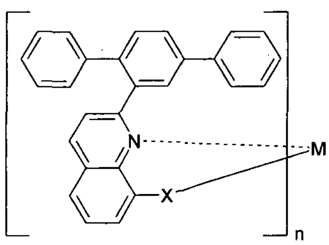

formule (B-9)

**10.** Composition comprenant un ou plusieurs composé(s) selon une ou plusieurs des revendications 1 à 9 et au moins une molécule fonctionnelle additionnelle qui est sélectionnée parmi le groupe qui est constitué par des émetteurs fluorescents, des émetteurs phosphorescents, des matériaux hôtes, des matériaux de matrice, des matériaux de transport d'électrons, des matériaux d'injection d'électrons, des matériaux conducteurs de trous, des matériaux d'injection de trous, des matériaux de blocage d'électrons, des matériaux de blocage de trous et des dopants de type n.

**11.** Composition selon la revendication 10, **caractérisée en ce que** le matériau fonctionnel additionnel est un matériau de transport d'électrons, lequel est de préférence sélectionné parmi les pyridines, les pyrimidines, les pyridazines, les pyrazines, les oxadiazoles, les oxazoles, les lactames, les quinolines, les quinoxalines, les anthracènes, les benzanthracènes, les pyrènes, les pérylènes, les benzimidazoles, les triazines, les cétones, les oxydes de phosphine et les phénazines, de façon très préférable parmi les triazines.

**12.** Composition selon la revendication 10 ou 11, **caractérisée en ce que** le matériau fonctionnel additionnel est un matériau de transport d'électrons qui comprend un composé de la formule générale (2) qui suit :

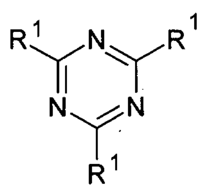

formule (2)

où ce qui suit s'applique aux symboles et indices utilisés :

$R^1$ est pour chaque occurrence, de manière identique ou différente, H, D, F, Cl, Br, I, CHO, $N(R^2)_2$, $N(Ar^1)_2$, $B(Ar^1)_2$, $C(=O)Ar^1$, $P(=O)(Ar^1)_2$, $S(=O)Ar^1$, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, un groupe alkyle, alkényle, alkynyle, alcoxy ou thioalcoxy enchaîne droite qui comporte 1 à 40 atome(s) de C ou un groupe alkyle, alkényle, alkynyle, alcoxy ou thioalcoxy ramifié ou cyclique qui comporte 3 à 40 atomes de C, lequel peut dans chaque cas être substitué par un radical ou plusieurs radicaux

$R^2$, où un ou plusieurs groupe(s) $CH_2$ non adjacents peut/peuvent être remplacé(s) par $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atome(s) de H peut/peuvent être remplacé(s) par D, F, Cl, Br, I, CN ou $NO_2$, ou un système de cycle aromatique ou hétéroaromatique qui comporte 5 à 60 atomes de cycle aromatique, lequel peut dans chaque cas être substitué par un radical ou plusieurs radicaux $R^2$, ou un groupe aryloxy ou hétéroaryloxy qui comporte 5 à 60 atomes de cycle aromatique, lequel peut être substitué par un radical ou plusieurs radicaux $R^2$, ou un combinaison de ces systèmes ; deux substituants $R^2$ adjacents ou plus ici peuvent également former un système de cycle monocyclique ou polycyclique, aliphatique ou aromatique l'un avec l'autre ou les uns avec les autres ;
$Ar^1$ est pour chaque occurrence, de manière identique ou différente, un système de cycle aromatique ou hétéroaromatique qui comporte 5 à 30 atomes de cycle aromatique, lequel peut être substitué par un radical ou plusieurs radicaux $R^2$ ; deux radicaux $Ar^1$ qui sont liés au même atome d'azote, de phosphore ou de bore peuvent également être liés l'un à l'autre ici au moyen d'une liaison simple ou d'un pont qui est sélectionné parmi $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ et $P(=O)R^2$ ;
$R^2$ est pour chaque occurrence, de manière identique ou différente, H, D ou un radical hydrocarbone aliphatique, aromatique et/ou hétéroaromatique qui comporte 1 à 20 atome(s) de C, où, en outre, des atomes de H peuvent être remplacés par D ou F ; deux substituants $R^2$ adjacents ou plus ici peuvent également former un système de cycle monocyclique ou polycyclique, aliphatique ou aromatique l'un avec l'autre ou les uns avec les autres.

**13.** Composition selon la revendication 10, **caractérisée en ce que** le matériau fonctionnel additionnel est un dopant de type n.

**14.** Formulation comprenant au moins un composé selon une ou plusieurs des revendications 1 à 9 ou au moins une composition selon une ou plusieurs des revendications 10 à 13 et au moins un solvant.

**15.** Utilisation d'au moins un composé selon une ou plusieurs des revendications 1 à 9 ou d'au moins une composition selon une ou plusieurs des revendications 10 à 13 dans un dispositif électronique, de façon préférable dans un dispositif électroluminescent organique, de façon très préférable dans une diode émettrice de lumière organique (OLED) ou dans une cellule électrochimique émettrice de lumière organique (OLEC, LEC ou également LEEC), de façon très particulièrement préférable dans une OLED.

**16.** Dispositif contenant au moins un composé selon une ou plusieurs des revendications 1 à 9 ou au moins une composition selon une ou plusieurs des revendications 10 à 13.

**17.** Dispositif selon la revendication 16, **caractérisé en ce que** le dispositif est un dispositif électronique.

**18.** Dispositif selon la revendication 16 ou 17, **caractérisé en ce que** le dispositif est un dispositif électronique qui est sélectionné parmi le groupe qui est constitué par des dispositifs électroluminescents organiques, des circuits intégrés organiques, des transistors à effet de champ organiques, des transistors à film mince organiques, des cellules solaires organiques, des détecteurs optiques organiques, des photorécepteurs organiques et des dispositifs à extinction de champ organiques, où les dispositifs électroluminescents organiques ont la préférence.

**19.** Dispositif selon une ou plusieurs des revendications 16 à 18, **caractérisé en ce qu'**il s'agit d'un dispositif électroluminescent organique qui est sélectionné parmi le groupe qui est constitué par les diodes émettrices de lumière organiques (OLED), les transistors émetteurs de lumière organiques, les cellules électrochimiques émettrices de lumière organiques (OLEC, LEC ou également LEEC) et les diodes laser organiques, de façon préférable parmi les OLED et les OLEC et de façon très préférable parmi les OLED.

**20.** Dispositif selon une ou plusieurs des revendications 16 à 19, **caractérisé en ce qu'**il contient l'au moins un composé selon une ou plusieurs des revendications 1 à 9 ou l'au moins une composition selon une ou plusieurs des revendications 10 à 13 dans une couche conductrice d'électrons.

**21.** Dispositif selon une ou plusieurs des revendications 16 à 19, **caractérisé en ce qu'**il contient l'au moins un composé selon une ou plusieurs des revendications 1 à 9 ou l'au moins une composition selon une ou plusieurs des revendications 10 à 13 dans une couche d'injection d'électrons ou dans une couche de transport d'électrons.

**22.** Procédé pour la préparation du composé selon une ou plusieurs des revendications 1 à 9 au moyen de la préparation du ligand sans métal et au moyen d'une réaction qui suit du ligand avec un sel métallique de manière à obtenir le complexe métallique selon une ou plusieurs des revendications 1 à 9.

**23.** Dispositif électroluminescent, de façon préférable une OLED ou une OLEC, contenant au moins un composé selon une ou plusieurs des revendications 1 à 9 ou au moins une composition selon une ou plusieurs des revendications 10 à 13 pour une utilisation en médecine.

**24.** Utilisation cosmétique d'un dispositif électroluminescent, de façon préférable une OLED ou une OLEC, contenant au moins un composé selon une ou plusieurs des revendications 1 à 9 ou au moins une composition selon une ou plusieurs des revendications 10 à 13.

Abbildung 1

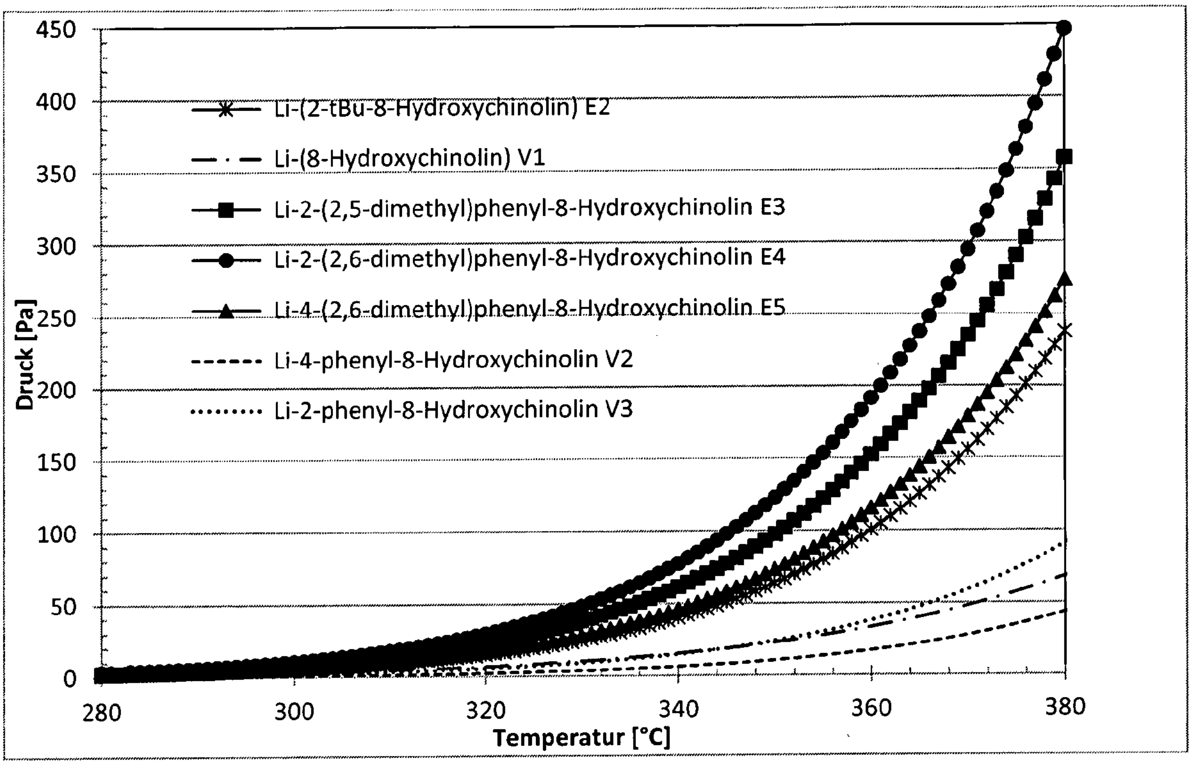
450
400
350
300
250
200
150
100
50
0
Druck [Pa]
280
300
320
340
360
380
Temperatur [°C]
Li-(2-tBu-8-Hydroxychinolin) E2
Li-(8-Hydroxychinolin) V1
Li-2-(2,5-dimethyl)phenyl-8-Hydroxychinolin E3
Li-2-(2,6-dimethyl)phenyl-8-Hydroxychinolin E4
Li-4-(2,6-dimethyl)phenyl-8-Hydroxychinolin E5
Li-4-phenyl-8-Hydroxychinolin V2
Li-2-phenyl-8-Hydroxychinolin V3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9013148 A1
- JP 2007063489 A
- WO 2010108579 A
- US 7294849 B
- WO 2010072300 A1
- US 6229012 B
- US 6225467 B
- WO 05053055 A
- DE 102008036982
- WO 200586251 A2
- WO 2012175535 A1
- WO 200900237 A1
- WO 2012168358 A1
- US 20070145355 A1
- EP 2452946 A1
- EP 2463927 A1
- WO 2012031735 A1
- EP 1837926 A1
- WO 2007107306 A1
- WO 2005011013 A
- WO 2004058911 A


### In der Beschreibung aufgeführte Nicht-Patentliteratur


- **M. S. ARNOLD et al.** *Appl. Phys. Lett.,* 2008, vol. 92, 053301
- *Tetrahedron Asymmetry,* 2001, vol. 12, 1345
- *J. Am. Chem. Soc.,* 2010, vol. 132, 13194
- *Nature,* 2012, vol. 492, 95
- *J. Am. Chem. Soc,* 2010, vol. 132, 13194
- *Monatshefte für Chemie,* 1991, vol. 122 (11), 935-41
- *J-Pestic. Sci.,* 1982, vol. 13, 161-168